(19) Europäisches Patentamt
European Patent Office
Office européen des brevets

(11) EP 4 317 193 A1

(12) EUROPEAN PATENT APPLICATION
published in accordance with Art. 153(4) EPC

(43) Date of publication:
**07.02.2024 Bulletin 2024/06**

(21) Application number: **22781210.4**

(22) Date of filing: **31.03.2022**

(51) International Patent Classification (IPC):
***C08B 31/12*** *(2006.01)* ***C08H 99/00*** *(2010.01)*

(52) Cooperative Patent Classification (CPC):
**C08B 31/12; C08H 99/00;** Y02W 30/62

(86) International application number:
**PCT/JP2022/016403**

(87) International publication number:
**WO 2022/211000 (06.10.2022 Gazette 2022/40)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **31.03.2021 JP 2021061021**

(71) Applicants:
- **Nagase & Co., Ltd.**
  **Osaka-shi, Osaka 550-8668 (JP)**
- **Nagase ChemteX Corporation**
  **Osaka-shi, Osaka 550-8668 (JP)**
- **Hayashibara Co., Ltd.**
  **Okayama-shi, Okayama 7028006 (JP)**

(72) Inventors:
- **OTA, Kusuo**
  **Tokyo 103-8355 (JP)**
- **NOZAKI, Takahiro**
  **Tokyo 103-8355 (JP)**
- **TANAKA, Atsushi**
  **Tokyo 103-8355 (JP)**
- **HOSOMI, Tetsuya**
  **Tatsuno-shi, Hyogo 679-4124 (JP)**
- **NISHIMOTO, Tomoyuki**
  **Okayama-shi, Okayama 702-8006 (JP)**
- **MIYATA, Manabu**
  **Okayama-shi, Okayama 702-8006 (JP)**

(74) Representative: **Mewburn Ellis LLP**
**Aurora Building**
**Counterslip**
**Bristol BS1 6BX (GB)**

# (54) METHOD FOR PRODUCING WATER-SOLUBLE POLYMER AND METHOD FOR PRODUCING WATER-ABSORBING RESIN

(57) The present invention provides a method for producing a water-soluble polymer with high production efficiency, and a method for producing a water-absorbing resin having excellent water absorption performance. The present invention relates to a method for producing a water-soluble polymer, comprising: (a1) reducing the molecular weight of a starch to obtain a partially degraded starch; and (a2) introducing an ionic functional group into the partially degraded starch obtained in the step (a1).

EP 4 317 193 A1

## Description

TECHNICAL FIELD

The present invention relates to a method for producing a water-soluble polymer, and a method for producing a water-absorbing resin.

BACKGROUND ART

Water-absorbing resins are widely used in various fields such as hygiene products, foods, agriculture and forestry, and civil engineering. As such water-absorbing resins, partially neutralized salts of polyacrylic acids or polymethacrylic acids are widely used, and water-absorbing resins made from polysaccharides such as starch are known.
Patent Literature 1 discloses a method of producing a water-absorbing resin by heat drying a carboxyalkylated starch to crosslink one starch with another. This document describes that it is preferred to reduce the decrease in the molecular weight of the starch during the carboxyalkylation reaction.
Patent Literature 2 discloses a method of producing a water-absorbing resin by subjecting carboxyalkylated polysaccharide particles to surface treatment with a non-crosslinking acid such as hydrochloric acid followed by heat drying or reaction with a crosslinking agent to crosslink one polysaccharide with another.
Patent Literature 3 discloses a method of producing a water-absorbing material by reacting a starch with a polybasic acid anhydride in an extruder. This document describes that it is preferred to reduce the decrease in the molecular weight of the starch during the reaction with the polybasic acid anhydride.

CITATION LIST

- Patent Literature

Patent Literature 1: U.S. Pat. No. 5,079,354
Patent Literature 2: JP 2010-504414 T
Patent Literature 3: JP 2007-222704 A

SUMMARY OF INVENTION

- Technical Problem

Conventional water-absorbing resins made from polysaccharides have not had sufficient water absorption performance. Moreover, since they have been produced from high-molecular weight polysaccharides, they have had an operability problem during the production due to the highly viscous raw materials. An object of the present invention is to provide a method for producing a water-soluble polymer with high production efficiency and a method for producing a water-absorbing resin having excellent water absorption performance.

- Solution to Problem

The present inventors have focused on the molecular weight of a starch to be used as a raw material for a water-absorbing resin, and have completed the present invention.
Specifically, the present invention relates to a method for producing a water-absorbing resin, including:

(c1) producing a water-soluble polymer by:

(a1) reducing a molecular weight of a starch to obtain a partially degraded starch; and
(a2) introducing an acidic group into the partially degraded starch obtained in the step (a1);

(c2) neutralizing the acidic group introduced into the water-soluble polymer; and
(c3) crosslinking one water-soluble polymer with another.

The acidic group is preferably an acidic group having a carboxyalkyl group, a carboxyalkenyl group, or a sulfoalkyl group.

The obtained water-absorbing resin preferably has the following characteristics:

(a) a water absorption rate under no pressure for ion-exchanged water is 100 to 400 g/g;
(b) a water retention rate for ion-exchanged water is 80 to 300 g/g;
(c) a water absorption rate under no pressure for physiological saline is 20 to 70 g/g; and/or
(d) a water retention rate for physiological saline is 7 to 60 g/g.

The obtained water-absorbing resin preferably has a ratio (A/B) of a water absorption rate under no pressure for ion-exchanged water (A) to a water absorption rate under no pressure for physiological saline (B) of 7 or less.
No crosslinking agent is preferably used in the step (c3) .
The present invention also relates to a method for degrading a water-absorbing resin, including subjecting the water-absorbing resin obtained by the above-described method to alkali treatment.
The present invention also relates to an article containing the water-absorbing resin obtained by the above-described method.
The present invention also relates to a method for producing a water-soluble polymer, including:

(a1) reducing a molecular weight of a starch to obtain a partially degraded starch; and
(a2) introducing an acidic group into the partially degraded starch obtained in the step (a1).

The partially degraded starch obtained in the step (a1) preferably has a weight average molecular weight (Mw) of 7,500,000 or less and/or a dispersity (weight average molecular weight (Mw)/number average molecular weight (Mn)) of 5 or more.
The present invention also relates to a method for producing a water-soluble polymer, including:

(b1) introducing an acidic group into a starch; and
(b2) reducing a molecular weight of the starch having the acidic group obtained in the step (b1) to obtain a partially degraded starch having the acidic group.

In the step (a1) or (b2), the molecular weight of the starch is preferably reduced by enzymatic treatment.
The obtained water-soluble polymer preferably has a weight average molecular weight (Mw) of 500,000 to 50,000,000 as determined by aqueous size exclusion chromatography using pullulan standards.
The present invention also relates to a method for producing a resin composition for producing a water-absorbing resin containing the water-soluble polymer obtained by the above-described method.

- Advantageous Effects of Invention

The method for producing a water-soluble polymer of the present invention can reduce the viscosity of an intermediate product and improve production efficiency. Moreover, the method for producing a water-absorbing resin of the present invention can produce a water-absorbing resin having excellent water absorption performance.

## DESCRIPTION OF EMBODIMENTS

### <<Method 1 for producing water-soluble polymer>>

A method for producing a water-soluble polymer of the present invention is characterized by including: (a1) reducing the molecular weight of a starch to obtain a partially degraded starch; and (a2) introducing an ionic functional group into the partially degraded starch obtained in the step (a1).

### <Step (a1) of reducing the molecular weight of a starch to obtain a partially degraded starch>

In this step, the molecular weight of a starch is reduced to reduce the viscosity. The type of starch as a raw material is not limited, and examples include waxy corn starch, tapioca starch, potato starch, corn starch (including waxy corn starch and high amylose starch), wheat starch, rice starch, and sweet potato starch.
The method for reducing the molecular weight of a starch is not limited, and examples include enzymatic treatment, acid treatment, physical crushing, etc. These methods may also be used in combination. In these methods, it is preferred to partially hydrolyze the glucoside bonds of the $\alpha$-glucose molecules constituting a starch, although the position and mode of degradation are not limited. The reactor used may be a reaction vessel, an extruder, etc.
When the molecular weight of a starch is reduced by enzymatic treatment, the enzyme to be used is preferably,

but not limited to, an endo-enzyme in order to efficiently reduce the molecular weight. Specific examples of the enzyme include α-amylase, cyclomaltodextrin glucanotransferase, 4-α-glucanotransferase, 4,6-α-glucanotransferase, amylomaltase, neopullulanase, and amylopullulanase, etc. These enzymes may be used in combination. The pH during the enzymatic treatment is not limited, but it is preferably pH 5.0 to 7.0. The pH can be adjusted by adding hydrochloric acid, acetic acid, sodium hydroxide, potassium hydroxide, or the like. The enzymatic treatment is preferably performed while heating and kneading the starch at 70°C to 110°C to gelatinize it. The enzymatic treatment may be performed after or at the same time as the gelatinization of the starch. Examples of the method for performing the enzymatic treatment after the gelatinization of the starch include a method including first suspending a starch in water and heating it to gelatinize it, followed by adding an enzyme thereto to perform an enzymatic reaction. Examples of the method for performing the enzymatic treatment at the same time as the gelatinization of the starch include a method including suspending a starch in water and adding an enzyme thereto to give a liquid mixture, followed by heating the liquid mixture within a temperature range that does not completely inactivate the enzyme.

When the molecular weight of a starch is reduced by acid treatment, specific examples of the acid to be used include, but are not limited to, hydrochloric acid, sulfuric acid, oxalic acid, acetic acid, formic acid, and trifluoroacetic acid, etc. The temperature during the acid treatment is preferably 150°C to 160°C.

When a starch is partially degraded by physical crushing, specific means include radiation, shearing, trituration, high-pressure treatment, ultrasonication, heat degradation, photodegradation, and combinations thereof.

If the weight average molecular weight of the partially degraded starch obtained in the step (a1) is high, the reaction at the time of the introduction of an ionic functional group or the operability in the purification step tends to be reduced due to the increased viscosity. Therefore, the weight average molecular weight is preferably 7,500,000 or less, more preferably 5,000,000 or less. The lower limit of the weight average molecular weight of the partially degraded starch is not limited, but it is preferably 50,000 or more, more preferably 200,000 or more. If the weight average molecular weight is less than 50,000, the water retention properties of the water-absorbing resin tend to be reduced. Here, the method for measuring the weight average molecular weight is not limited. For example, the weight average molecular weight can be determined by aqueous size exclusion chromatography relative to a calibration curve of molecular weight vs. elution time prepared using pullulans having known molecular weights.

The number average molecular weight of the partially degraded starch obtained in the step (a1) is not limited, but in view of the viscosity it is preferably 1,000,000 or less. Moreover, the lower limit of the number average molecular weight of the partially degraded starch is preferably 10,000 or more, more preferably 50,000 or more. Here, the method for measuring the number average molecular weight is not limited. For example, the number average molecular weight can be determined by aqueous size exclusion chromatography relative to a calibration curve of molecular weight vs. elution time prepared using pullulans having known molecular weights.

The weight average molecular weight or number average molecular weight of the partially degraded product obtained in the step (a1) may also be controlled by mixing two or more partially degraded starches. In this case, the weight average molecular weight or number average molecular weight of the mixture preferably satisfies the numerical range described above.

The dispersity (weight average molecular weight/number average molecular weight) of the partially degraded starch is not limited, but it is preferably 5 or more, more preferably 7 or more. The upper limit of the dispersity is not limited as long as the numerical range of the weight average molecular weight described above is satisfied, but it is usually 70 or less.

<Step (a2) of introducing an ionic functional group into the partially degraded starch>

In this step, the partially degraded starch obtained in the step (a1) is reacted with an ionic functional group-containing compound. The ionic functional group is not limited as long as it can impart crosslinkability to the partially degraded starch, and it may be either an acidic group or a basic group.

Examples of the acidic group include an acidic group having a carboxyl group such as a carboxyalkyl group or a carboxyalkenyl group; an acidic group having a sulfo group such as a sulfoalkyl group or a sulfoalkenyl group; and an acidic group having a phospho group such as a phosphoalkyl group or a phosphoalkenyl group.

The carboxyalkyl group is an alkyl group substituted with a carboxyl group. The number of carbon atoms of the alkyl group to be substituted with a carboxyl group is preferably 1 to 8, more preferably 1 to 5. The alkyl group may be either linear or branched. Specific examples of the alkyl group include a methyl group, an ethyl group, an n-propyl group, an i-propyl group, an n-butyl group, an i-butyl group, an s-butyl group, a t-butyl group, an n-pentyl group, a 1-methyl-n-butyl group, a 2-methyl-n-butyl group, a 3-methyl-n-butyl group, a 1,1-dimethyl-n-propyl group, a 1,2-dimethyl-n-propyl group, a 2,2-dimethyl-n-propyl group, and a 1-ethyl-n-propyl group.

Specific examples of the carboxyalkyl group include a carboxymethyl group, a carboxyethyl group, a carboxypropyl group, a carboxybutyl group, and a carboxypentyl group.

The carboxyalkenyl group is an alkenyl group substituted with a carboxyl group. The number of carbon atoms

of the alkenyl group to be substituted with a carboxyl group is preferably 2 to 8, more preferably 2 to 4. The alkenyl group may be either linear or branched. Specific examples of the alkenyl group include an ethenyl group, a 1-propenyl group, a 2-propenyl group, a 1-methyl-1-ethenyl group, a 1-butenyl group, a 2-butenyl group, a 3-butenyl group, a 2-methyl-1-propenyl group, a 2-methyl-2-propenyl group, a 1-ethylethenyl group, 1-methyl-1-propenyl group, and a 1-methyl-2-propenyl group.

Specific examples of the carboxyalkenyl group include a carboxyethenyl group, a carboxypropenyl group, and a carboxybutenyl group.

The sulfoalkyl group is an alkyl group substituted with a sulfo group. Examples of the alkyl group to be substituted with a sulfo group include the alkyl groups listed for the carboxyalkyl group. Specific examples of the sulfoalkyl group include a sulfomethyl group, a sulfoethyl group, and a sulfopropyl group.

The sulfoalkenyl group is an alkenyl group substituted with a sulfo group. Examples of the alkenyl group to be substituted with a sulfo group include the alkenyl groups listed for the carboxyalkenyl group. Specific examples of the sulfoalkenyl group include a sulfoethenyl group, and a sulfopropenyl group.

The phosphoalkyl group is an alkyl group substituted with a phospho group. Examples of the alkyl group to be substituted with a phospho group include the alkyl groups listed for the carboxyalkyl group. Specific examples of the phosphoalkyl group include a phosphomethyl group, a phosphoethyl group, and a phosphopropyl group.

The phosphoalkenyl group is an alkenyl group substituted with a phospho group. Examples of the alkenyl group to be substituted with a phospho group include the alkenyl groups listed for the carboxyalkenyl group. Specific examples of the phosphoalkenyl group include a phosphoethenyl group and a phosphopropenyl group.

The acidic group is preferably an acidic group having a carboxyl group or a sulfo group, more preferably a carboxyalkyl group, a carboxyalkenyl group, or a sulfoalkyl group, even more preferably a carboxyalkyl group having 1 to 5 carbon atoms, from the standpoint of ease of introduction into the partially degraded starch.

In order to introduce an acidic group, the partially degraded starch may be reacted with an acidic group-containing compound or a precursor thereof. The acidic group-containing compound is not limited as long as it can introduce the acidic group described above. Examples include a haloalkyl compound having an acidic group, a haloalkenyl compound having an acidic group, an acid anhydride, and salts thereof. Examples of the halogen constituting the haloalkyl compound or haloalkenyl compound include chlorine and bromine.

Specific examples of the acidic group-containing compound include monochloroacetic acid, monobromoacetic acid, 3-bromopropionic acid, 6-bromohexanoic acid, succinic anhydride, maleic anhydride, vinylsulfonic acid, phosphorus oxychloride, ethyl monochloroacetate, and sodium or potassium salts thereof. Specific examples of the salts include sodium monochloroacetate, potassium monochloroacetate, and sodium vinylsulfonate.

Examples of the precursor of the acidic group-containing compound include acrylonitrile. Examples of the method using acrylonitrile include a method including first reacting acrylonitrile with a starch or a partially degraded product thereof under basic conditions to introduce a cyanoethyl group, deriving an amide group from the cyanoethyl group (Synthesis; 1989(12):949-950), and subjecting the resulting amide to alkaline hydrolysis.

Specific examples of the basic group include a basic group having a primary amino group, a secondary amino group, a tertiary amino group, a quaternary ammonium group, or the like.

Among these groups, the group is preferably an acidic group, more preferably an acidic group having a carboxyl group, even more preferably a carboxyalkyl group, from the standpoint of ease of introduction into the partially degraded starch.

These ionic functional groups may be introduced as follows. When an acidic group is to be introduced, a starch or a partially degraded product thereof may be reacted with an acidic group-containing compound or a precursor thereof.

Specific examples of the method for introducing an acidic group include: for a sulfo group, a method including reaction with vinylsulfonic acid or sodium vinylsulfonate under basic conditions; and for a phospho group, a method including reaction with phosphorus oxychloride under basic conditions. Examples of the method for introducing a basic group include a method using glycidyltrimethylammonium chloride or epichlorohydrin and an amine.

The reaction of the partially degraded starch with monochloroacetic acid to produce a water-soluble polymer is summarized in formula (I).

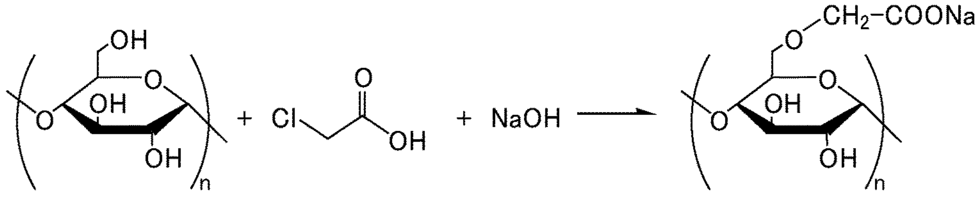

( I )

The reaction of the partially degraded starch with 3-bromopropionic acid to produce a water-soluble polymer

is summarized in formula (II).

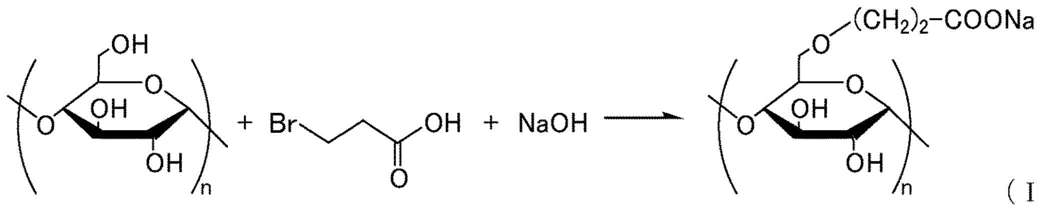

(II)

The reaction of the partially degraded starch with 6-bromohexanoic acid to produce a water-soluble polymer is summarized in formula (III).

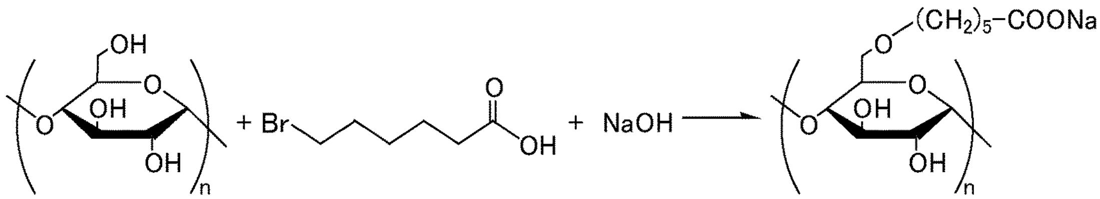

(III)

The reaction of the partially degraded starch with succinic anhydride to produce a water-soluble polymer is summarized in formula (IV).

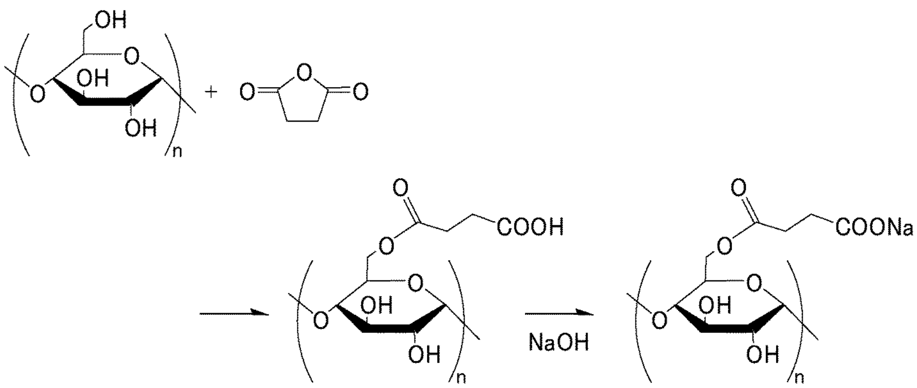


(IV)

The reaction of the partially degraded starch with maleic anhydride to produce a water-soluble polymer is summarized in formula (V).

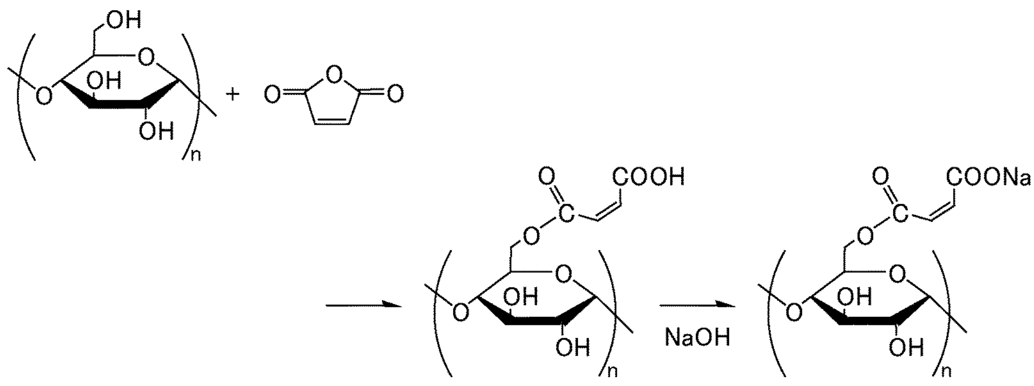


(V)

The reaction of the partially degraded starch with sodium vinylsulfonate to produce a water-soluble polymer is summarized in formula (VI).

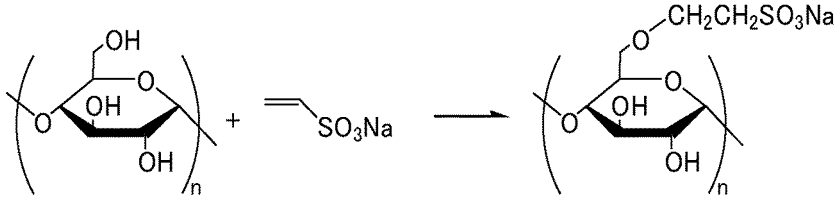

(VI)

Formulas (I) to (VI) show water-soluble polymers in which a sodium salt of an acidic group is introduced into all hydroxyl groups at the 6-position of glucose units, but there may remain a hydroxyl group with no acidic group introduced thereinto. There may also be an acidic group that has not been neutralized by a salt. The acidic group can be introduced into a hydroxyl group at any position as long as the hydroxyl group is present in the partially degraded starch, and the hydroxyl group may be the hydroxyl group at any of the 1-, 2-, 3-, 4-, and 6-positions.

The reaction between the partially degraded starch and the ionic functional group-containing compound may be performed under any condition, but is preferably performed under basic conditions. When a haloalkyl compound or a haloalkenyl compound is used as the ionic functional group-containing compound, 1 to 1.5 equivalents of an alkaline agent is preferably used relative to the haloalkyl compound or haloalkenyl compound. Examples of the alkaline agent include sodium hydroxide, potassium hydroxide, lithium hydroxide, ammonia, sodium carbonate, and potassium carbonate.

The ionic functional group introduced into the partially degraded starch preferably forms a salt with the sodium, potassium, lithium, ammonia, or the like derived from the alkaline agent. Therefore, the alkaline agent is preferably used in an amount required for both the reaction of the haloalkyl compound or haloalkenyl compound with the partially degraded starch and the neutralization of the acidic group of the haloalkyl compound or haloalkenyl compound. For example, when chloroacetic acid is used as the ionic functional group-containing compound, the alkaline agent is preferably used theoretically in an amount of 2 equivalents or more relative to chloroacetic acid. When sodium chloroacetate is used, the alkaline agent is preferably used in an amount of 1 equivalent or more relative to sodium chloroacetate, since the acidic group has previously been neutralized.

The amount of the ionic functional group-containing compound to be used can be arbitrarily set depending on the desired total acid value (degree of etherification) of the water-soluble polymer. The amount is usually preferably 0.5 to 5.0 equivalents, more preferably 0.5 to 2.0 equivalents, per mol of hydroxyl groups in the partially degraded starch. When an aqueous solution with a haloalkyl compound such as chloroacetic acid is to be reacted, the haloalkyl compound is required in excess of the theoretical amount because the introduction reaction of an acidic group will compete with the hydrolysis reaction of the haloalkyl compound. The amount of the haloalkyl compound to be used in the reaction of the aqueous solution is preferably set at 5 equivalents or less relative to the theoretical value.

The temperature during the reaction between the partially degraded starch and the ionic functional group-containing compound is not limited, but it is preferably 0°C to 120°C. The reaction time is not limited, but it is preferably 1 to 24 hours. The reaction may be performed in water, but it may be performed in a solvent mixture of water with an alcohol such as methanol, ethanol, isopropanol, butanol, ethylene glycol, diethylene glycol, propylene glycol, or ethylene glycol monoethyl ether, a glycol ether such as ethylene glycol dimethyl ether, or other solvent. The reaction may also be performed by dispersing the powder of the dried partially degraded starch in a hydrophilic solvent such as an alcohol

such as methanol, ethanol, isopropanol, or butanol, or a glycol ether such as ethylene glycol dimethyl ether. When a solvent mixture is used, the proportion of the solvent other than water is preferably 50% by volume or less based on the solvent mixture. The reactor used may be a reaction vessel, an extruder, etc.

When a haloalkyl compound, such as chloroacetic acid, or a salt thereof is used as an acidic group-containing compound, the temperature during the reaction thereof with the partially degraded starch is not limited, but it is preferably 0°C to 100°C.

In particular, when chloroacetic acid or a salt thereof is used as the haloalkyl compound, the reaction is preferably performed at 25°C to 90°C to prevent hydrolysis due to water in the reaction solution. The reaction time is preferably a period of time until the haloalkyl compound used as a raw material is consumed, more preferably 1 to 12 hours for stability of the haloalkyl compound and process efficiency. The reaction may be performed in water, but it may be performed in a solvent mixture of water with an alcohol such as methanol, ethanol, isopropanol, or butanol, a glycol ether such as ethylene glycol dimethyl ether, or other solvent. The reaction may also be performed by dispersing the powder of the dried partially degraded starch in a hydrophilic solvent such as an alcohol such as methanol, ethanol, isopropanol, or butanol, or a glycol ether such as ethylene glycol dimethyl ether. When a solvent mixture is used, the proportion of the solvent other than water is preferably 50% by volume or less based on the solvent mixture. The reactor used may be a reaction vessel, an extruder, etc.

Moreover, when an acid anhydride is used as the ionic functional group-containing compound, the reaction is allowed to proceed only by mixing and heating the partially degraded starch and the acid anhydride. However, in order to promote the reaction, a catalyst may be used, such as sodium carbonate, sodium hydroxide, a tertiary amine such as triethylamine, an imidazole such as 2-methylimidazole, a quaternary ammonium salt such as tetrabutylammonium bromide, or a phosphonium salt such as tetrabutylphosphonium bromide. The amount of such a catalyst to be added is preferably 0.1 equivalents or less relative to the ionic functional group-containing compound. These catalysts may be used alone or in combinations of two or more.

The reaction time is preferably a period of time until the acid anhydride used as a raw material is consumed, more preferably 1 to 12 hours. The end point of the reaction can be determined by measurement of the acid value or IR measurement. The reaction may be performed in water, but the reaction solvent is preferably an aprotic solvent such as dimethylsulfoxide, dimethylformamide, dimethylacetamide, or N-methylpyrrolidone, in order to prevent hydrolysis or alcoholysis of the acid anhydride. When a solvent mixture is used, the proportion of the solvent other than water is preferably 50% by volume or more based on the solvent mixture. When the reaction is performed with no solvent, the reaction is preferably performed at a temperature equal to or more than the melting point of the acid anhydride because the acid anhydride can serve as a solvent. When the reaction is performed with a solvent, the reaction temperature is preferably 50°C to 100°C, more preferably 70°C to 90°C. The reactor used may be a reaction vessel, an extruder, etc.

<<Method 2 for producing water-soluble polymer>>

A method for producing a water-soluble polymer of the present invention is characterized by including: (b1) introducing an ionic functional group into a starch; and (b2) reducing the molecular weight of the starch having the ionic functional group obtained in the step (b1) to obtain a partially degraded starch having the ionic functional group.

<Step (b1) of adding an ionic functional group to a starch>

In this step, a starch is reacted with an ionic functional group-containing compound. The starch, ionic functional group, and ionic functional group-containing compound used are as described above for the step (a2) of introducing an ionic functional group into the partially degraded starch.

The conditions for the reaction between the starch and the ionic functional group-containing compound are not limited, but when a haloalkyl compound is used as the ionic functional group-containing compound, an alkaline agent is preferably used in an amount of 1 to 1.5 equivalents relative to the haloalkyl compound. The pH is preferably pH 10.5 to 12.5, more preferably pH 11 to 12, in order to stabilize the starch. Examples of the alkaline agent used for pH adjustment include sodium hydroxide, potassium hydroxide, lithium hydroxide, ammonia, sodium carbonate, and potassium carbonate.

The ionic functional group introduced into the starch preferably forms a salt with the sodium, potassium, lithium, ammonia, or the like derived from the alkaline agent. Therefore, the alkaline agent is preferably used in an amount required for both the reaction of the haloalkyl compound with the starch and the neutralization of the acidic group of the haloalkyl compound. For example, when chloroacetic acid is used as the ionic functional group-containing compound, the alkaline agent is preferably used theoretically in an amount of 2 equivalents or more relative to chloroacetic acid. When sodium chloroacetate is used, the alkaline agent is preferably used in an amount of 1 equivalent or more relative to sodium chloroacetate, since the acidic group has previously been neutralized.

The amount of the ionic functional group-containing compound to be used can be arbitrarily set depending on

the desired total acid value (degree of etherification) of the water-soluble polymer. The amount is usually preferably 0.5 to 5.0 equivalents, more preferably 0.5 to 2.0 equivalents, per mol of hydroxyl groups in the starch. When an aqueous solution with a haloalkyl compound such as chloroacetic acid is to be reacted, the haloalkyl compound is required in excess of the theoretical amount because the introduction reaction of the acidic group competes with the hydrolysis reaction of the haloalkyl compound. The amount of the haloalkyl compound to be used in the reaction of the aqueous solution is preferably set at 5 equivalents or less over the theoretical value.

The temperature during the reaction between the starch and the ionic functional group-containing compound is not limited, but it is preferably 0°C to 120°C. The reaction time is not limited, but it is preferably 1 to 24 hours. The reaction may be performed in water, but it may be performed in a solvent mixture of water with an alcohol such as methanol, ethanol, isopropanol, butanol, ethylene glycol, diethylene glycol, propylene glycol, or ethylene glycol monoethyl ether, a glycol ether such as ethylene glycol dimethyl ether, or other solvent. When a solvent mixture is used, the proportion of the solvent other than water is preferably 50% by volume or less based on the solvent mixture. The reactor used may be a reaction vessel, an extruder, etc.

When a haloalkyl compound such as chloroacetic acid is used as an acidic group-containing compound, the temperature during the reaction thereof with the starch is not limited, but it is preferably 0°C to 100°C.

In particular, when chloroacetic acid or a salt thereof is used as the haloalkyl compound, the reaction is preferably performed at 25°C to 60°C to prevent hydrolysis due to the water in the reaction solution. The reaction time is preferably a period of time until the haloalkyl compound used as a raw material is consumed, more preferably 1 to 6 hours from the standpoint of stability of the haloalkyl compound and process efficiency. The reaction may be performed in water, but it may be performed in a solvent mixture of water with an alcohol such as methanol, ethanol, isopropanol, or butanol, a glycol ether such as ethylene glycol dimethyl ether, or other solvent. The reaction may also be performed by dispersing the powder of the dried partially degraded starch in a hydrophilic solvent such as an alcohol such as methanol, ethanol, isopropanol, or butanol, or a glycol ether such as ethylene glycol dimethyl ether. When a solvent mixture is used, the proportion of the solvent other than water is preferably 50% by volume or less based on the solvent mixture. The reactor used may be a reaction vessel, an extruder, etc.

<Step (b2) of reducing the molecular weight of the starch having the ionic functional group to obtain a partially degraded starch having the ionic functional group>

In this step, the molecular weight of the starch having the ionic functional group obtained in the step (b1) is reduced to reduce the viscosity. The molecular weight of the starch may be reduced by any method, such as by subjecting the starch to enzymatic treatment, acid treatment, physical crushing, etc. These methods are as described for (a1) Step of reducing the molecular weight of a starch to obtain a partially degraded starch.

<<Physical properties of water-soluble polymer>>

The molecular weight of the water-soluble polymer produced by the present invention is not limited, but the water-soluble polymer preferably has a weight average molecular weight of 500,000 to 50,000,000, more preferably 500,000 to 46,000,000, even more preferably 700,000 to 20,000,000 as determined by aqueous size exclusion chromatography using pullulan standards. If the weight average molecular weight is less than 500,000, the water retention properties of the water-absorbing resin tend to be reduced, whereas if it is more than 50,000,000, the water absorption performance of the water-absorbing resin tends to be reduced. Here, the weight average molecular weight as determined by aqueous size exclusion chromatography using pullulan standards can be determined by aqueous size exclusion chromatography relative to a calibration curve of molecular weight vs. elution time prepared using pullulans having known molecular weights.

When the ionic functional group is an acid group, the total acid value of the water-soluble polymer is preferably 50 to 350 mg KOH/g, more preferably 70 to 300 mg KOH/g. The total acid value means the acid value measured by returning the neutralized acidic group to an unneutralized state, which represents the total amount of acidic groups introduced into the water-soluble polymer. If the total acid value is less than 50 mg KOH/g or more than 350 mg KOH/g, the water-absorbing resin obtained after crosslinking the water-soluble polymer tends to have lower water absorption performance for an aqueous solution containing an electrolyte such as physiological saline.

When the acidic group on the water-soluble polymer has been introduced by reacting a haloalkyl compound having an acidic group with the hydroxyl group of a starch or a partially degraded product thereof, the amount of acidic groups introduced can also be represented by the degree of etherification. The degree of etherification of the water-soluble polymer is preferably 0.1 to 2.0, more preferably 0.2 to 1.5. The degree of etherification can be determined by, for example, an ashing-titration method. Moreover, since the total acid value is detected via the introduction of an acidic group, when the starch or partially degraded starch used as a raw material contains no acidic group, the acidic groups detected by the measurement of the total acid value are considered to be equivalent to those introduced by the etherification reaction. Therefore, when the starch or partially degraded starch as a raw material contains no acidic group, the degree of etherification may be calculated conveniently from the above total acid value measurement. For example, when carboxymethyl groups as acidic groups all have been neutralized into a sodium salt, the degree of etherification can be calculated as follows:

$$\text{Degree of etherification} = (162 \times \text{total acid value}) \div (56100 - 80 \times \text{total acid value}).$$

Here, the total acid value in this case is expressed in unit of mg KOH/g.

The free acid value of the water-soluble polymer is preferably 5 to 30 mg KOH/g, more preferably 7 to 25 mg KOH/g. The free acid value means the acid value measured for unneutralized acidic groups. If the free acid value is less than 5 mg KOH/g, the strength of the water-absorbing resin tends to be insufficient, whereas if it is more than 30 mg KOH/g, the water absorption performance tends to be reduced.

The dispersity (weight average molecular weight/number average molecular weight) of the water-soluble polymer is not limited, but it is preferably 5 to 110, more preferably 7 to 70. If the dispersity is less than 5 or more than 110, the water absorption performance of the water-absorbing resin tends to be reduced. The number average molecular weight of the water-soluble polymer is determined by aqueous size exclusion chromatography.

<<Method for producing water-absorbing resin>>

A method for producing a water-absorbing resin of the present invention is characterized by including: (c1) producing a water-soluble polymer by the above-described method; (c2) neutralizing the ionic functional group introduced into the water-soluble polymer; and (c3) crosslinking one water-soluble polymer with another.

<Step (c2) of neutralizing the ionic functional group introduced into the water-soluble polymer>

In this step, the salt of the ionic functional group added to the partially degraded starch is neutralized. Some salt-forming ionic functional groups can be converted to free ionic functional groups by neutralization. Neutralization is preferably performed on some ionic functional group salts. For example, when monochloroacetic acid is used as the above-described ionic functional group-containing compound and sodium hydroxide is used as the alkaline agent, a sodium salt of a carboxyl group will be added to the partially degraded starch. By adding an acid thereto, some carboxyl groups are converted to free carboxylic acids.

When the ionic functional group is an acidic group, an acid is used for neutralization. The acid is not limited, but when the acidic group is a carboxyl group, the acid is preferably an acid having a pKa equal to or less than that of the carboxyl group. Examples of such acids include hydrochloric acid, sulfuric acid, phosphoric acid, nitric acid, acetic acid, formic acid, and trichloroacetic acid. A strong acid is preferably used for neutralization of a sulfo group or phospho group. Examples of the strong acid to be used include mineral acids such as hydrochloric acid and sulfuric acid, and strongly acidic ion exchange resins. The neutralization can be performed with a known equipment such as a reaction vessel or an extruder. After the addition of the acid, stirring is preferably performed at 0°C to 50°C for 0.2 to 1 hour for the neutralization reaction. The neutralization reaction is preferably performed at pH 6.8 to 7.2.

When the ionic functional group is a basic group, an alkaline agent is used for neutralization. The alkaline agent is preferably, but not limited to, an alkaline agent having a pKa equal to or more than that of the basic group. Examples of such alkaline agents include sodium hydroxide and potassium hydroxide. The neutralization can be performed with a known equipment such as a reaction vessel or an extruder. After the addition of the alkaline agent, stirring is preferably performed at 0°C to 50°C for 0.2 to 1 hour for the neutralization reaction. The neutralization reaction is preferably performed at pH 6.8 to 7.2.

In the neutralization reaction, a salt may be formed between the halogen derived from the ionic functional group-containing compound and the metal or ammonia derived from the alkaline agent. Desalting may be performed by dropping an aqueous solution in which the water-soluble polymer is dissolved in water into a hydrophilic solvent such as methanol, ethanol, isopropanol, acetone, or acetonitrile, reprecipitating the water-soluble polymer, and collecting it by filtration, followed by redispersing and stirring the water-soluble polymer collected by filtration in hydrous methanol (with a water content of about 70 to 90%), and then collecting the particles of the water-soluble polymer by filtration, thereby washing them. Alternatively, desalting may be performed by treating an aqueous solution of the hydrophilic polymer with a filter having an ultrafiltration membrane. The washing liquid used for desalting may be water or a liquid mixture of water and a hydrophilic organic solvent such as methanol, ethanol, propanol, acetone, or acetonitrile. The desalting is preferably performed until the salt concentration in the water-soluble polymer reaches 1% or less.

<Step (c3) of crosslinking one water-soluble polymer with another>

In this step, one water-soluble polymer is crosslinked with another. The crosslinks formed here correspond to the internal crosslinks of the water-absorbing resin. The crosslinks can be formed with no crosslinking agent, for example, by heat drying the water-soluble polymer under hydrous conditions. The water-soluble polymer at the start of heat drying may be in the form of an aqueous solution or a wet powder containing a hydrous solvent having a moisture content of 1% by weight or more. When the wet powder containing the hydrous solvent is dried, the wet ratio of the wet powder at the start of drying is preferably 1 to 85% by weight, more preferably 20 to 80% by weight, even more preferably 55 to 75% by weight. As used herein, the term "wet ratio" refers to the proportion of the total amount of water and hydrophilic solvent in the wet powder. The temperature during the heating is preferably 50°C to 150°C, more preferably 60°C to 130°C. The drying may be performed by any method, such as by using a drum dryer, a spray dryer, a Nauta mixer, etc.

The crosslinks are preferably formed with the ionic functional group introduced into the water-soluble polymer in the step (c1). Examples of the crosslinked structure include ionic bonding between ionic functional groups and coordinate bonding with metal ions, and, when the ionic functional group is an acidic group having a carboxyl group, examples include hydrogen bonding (for example, dimerization of the carboxyl group). To facilitate the degradation of the resulting water-absorbing resin, the crosslinking is preferably not via covalent bonding. Examples of crosslinking via covalent bonding include an ester bond, an ether bond, a carbon-carbon single bond (C-C bond), and a carbon-carbon double bond (C=C bond).

A solvent other than water may be used in combination with water during the crosslinking. Examples of the solvent other than water include lower aliphatic alcohols such as methanol, ethanol, n-propanol, and isopropanol; ketones such as acetone; ethers such as dioxane, tetrahydrofuran, and methoxy (poly)ethylene glycol; and amides such as ε-caprolactam and N,N-dimethylformamide. The proportion of the solvent other than water to the total solvent is preferably adjusted depending on the boiling point of the solvent. When the boiling point of the solvent is 100°C or less, the proportion is preferably 70% by volume or more, whereas it is more than 100°C, the proportion is preferably 30% by volume or less.

As described above, the crosslinked structure can be formed with no crosslinking agent, but they may be formed with a crosslinking agent. Examples of the crosslinking agent include epoxy compounds, polyhydric alcohol compounds, polyamine compounds, polyisocyanate compounds, alkylene carbonate compounds, haloepoxy compounds, halohydrin compounds, polyvalent oxazoline compounds, carbodiimide compounds, silane coupling agents, and polyvalent metal compounds.

Examples of the epoxy compounds include glycidyl succinate, sorbitol polyglycidyl ether, trimethylolpropane polyglycidyl ether, polyethylene glycol diglycidyl ether, glycerol polyglycidyl ether, diglycerol polyglycidyl ether, polyglycerol polyglycidyl ether, propylene glycol diglycidyl ether, polypropylene glycol diglycidyl ether, and glycidol.

Examples of the polyhydric alcohol compounds include ethylene glycol, diethylene glycol, propylene glycol, triethylene glycol, tetraethylene glycol, polyethylene glycol, 1,3-propanediol, dipropylene glycol, 2,2,4-trimethyl-1,3-pentanediol, polypropylene glycol, glycerin, polyglycerin, 2-butene-1,4-diol, 1,3-butanediol, 1,4-butanediol, 1,5-pentanediol, 1,6-hexanediol, 1,2-cyclohexanedimethanol, 1,2-cyclohexanediol, trimethylolpropane, diethanolamine, triethanolamine, polyoxypropylene, oxyethylene-oxypropylene block copolymers, pentaerythritol, and sorbitol.

Examples of the polyamine compounds include ethylenediamine, diethylenetriamine, triethylenetetramine, tetraethylenepentamine, pentaethylenehexamine, polyethyleneimine, inorganic or organic salts of these polyamine compounds (such as azitinium salts), and polysaccharides having amino groups such as chitin.

Examples of the polyisocyanate compounds include 2,4-tolylene diisocyanate and hexamethylene diisocyanate, and examples of the polyvalent oxazoline compounds include 1,2-ethylenebisoxazoline.

Examples of the alkylene carbonate compounds include 1,3-dioxolan-2-one, 4-methyl-1,3-dioxolan-2-one, 4,5-dimethyl-1,3-dioxolan-2-one, 4,4-dimethyl-1,3-dioxolan-2-one, 4-ethyl-1,3-dioxolan-2-one, 4-hydroxymethyl-1,3-dioxolan-2-one, 1,3-dioxan-2-one, 4-methyl-1,3-dioxan-2-one, and 4,6-dimethyl-1,3-dioxan-2-one.

Examples of the haloepoxy compounds include epichlorohydrin, epibromohydrin, α-methylepichlorohydrin, and polyamine adducts thereof (for example, Kymene® available from Hercules Incorporated).

Other examples of known crosslinking agents that can be used include waterborne carbodiimide compounds (for example, CARBODILITE available from Nisshinbo Chemical Inc.); silane coupling agents such as γ-glycidoxypropyltrimethoxysilane and γ-aminopropyltriethoxysilane; and polyvalent metal compounds such as hydroxides or chlorides of zinc, calcium, magnesium, aluminum, iron, zirconium, etc.

The content of water and/or hydrophilic solvent in the water-absorbing resin obtained by crosslinking can be adjusted by subjecting the water-absorbing resin to a treatment such as heating or drying. The content of water and/or hydrophilic solvent in the water-absorbing resin is preferably 0.1 to 20%, more preferably 1 to 19%. If the content is less than 0.1%, the water absorption speed tends to decrease, whereas if it is more than 20%, the particles of the water-absorbing resin tend to aggregate (easily form clumps) upon absorption of water. Examples of the hydrophilic solvent include methanol, ethanol, n-propanol, isopropanol, acetone, ethylene glycol, propylene glycol, diethylene glycol, ethylene glycol monoethyl ether, ethylene glycol monobutyl ether, diethylene glycol dimethyl ether, and dimethylsulfoxide.

In the production of the water-absorbing resin, surface crosslinking may be performed in addition to the crosslinking in the step (c3). The surface crosslinking can improve the strength of the water-absorbing resin. Examples of the crosslinking agent used for surface crosslinking include crosslinking agents as described above for the crosslinking in the step (c3). Preferred among them are epoxy compounds, with ethylene glycol diglycidyl ether, sorbitol polyglycidyl ether, or glycidyl succinate being more preferred.

Surface crosslinks can be formed by spraying a surface crosslinking agent to the water-absorbing resin, and then mixing them by a known method with a cylindrical mixer, V-shaped mixer, ribbon mixer, screw mixer, twin-arm mixer, pulverizing kneader, etc., thereby causing crosslinking. A surfactant may be added as necessary during the spraying and mixing.

The produced water-absorbing resin may be subjected to a treatment such as desalting or washing to remove impurities and by-products. Desalting can be performed with a filter having a reverse osmosis membrane, for example. The washing liquid used may be water or a liquid mixture of water and a hydrophilic organic solvent such as methanol, ethanol, or propanol.

<<Water-absorbing resin>>

The water absorption rate under no pressure of the water-absorbing resin produced as described above is determined by measuring the absorbency for physiological saline or ion-exchanged water of the water-absorbing resin with no load applied thereto as described in EXAMPLES. The water-absorbing resin in a solid state preferably has a water absorption rate under no pressure for ion-exchanged water of 100 to 400 g/g, more preferably 120 to 350 g/g. Moreover, the water absorption rate under no pressure for physiological saline is preferably 20 to 70 g/g, more preferably 30 to 65 g/g.

The water-absorbing resin preferably has a ratio (A/B) of the water absorption rate under no pressure for ion-exchanged water (A) to the water absorption rate under no pressure for physiological saline (B) of 7 or less, more preferably 5 or less.

The water retention rate of the water-absorbing resin is determined by measuring the absorbency for physiological saline or ion-exchanged water of the water-absorbing resin with a load of 150 G applied thereto as described in EXAMPLES. The water-absorbing resin in a solid state preferably has a water retention rate for ion-exchanged water of 80 to 300 g/g, more preferably 100 to 300 g/g. Moreover, the water retention rate for physiological saline is preferably 7 to 60 g/g, more preferably 10 to 60 g/g, even more preferably 20 to 60 g/g.

The water-absorbing resin may optionally contain a constitutional unit other than the water-soluble polymer described above. Examples of the constitutional unit other than the water-soluble polymer include polyacrylic acids (or salts thereof) such as crosslinked products of partially neutralized polyacrylic acids, self-crosslinking partially neutralized polyacrylic acids, and starch-acrylic acid graft polymers. Examples of the salts of acrylic acids include sodium salts, potassium salts, and ammonium salts. Other examples of the constitutional unit other than the water-soluble polymer include anionic unsaturated monomers and salts thereof such as methacrylic acid, maleic acid, vinylsulfonic acid, styrenesulfonic acid, 2-(meth)acrylamido-2-methylpropanesulfonic acid, 2-(meth)acryloylethanesulfonic acid, and 2-(meth)acryloylpropanesulfonic acid; nonionic unsaturated monomers containing hydrophilic groups such as acrylamide, methacrylamide, N-ethyl (meth)acrylamide, N-n-propyl (meth)acrylamide, N-isopropyl (meth)acrylamide, N,N-dimethyl (meth)acrylamide, 2-hydroxyethyl (meth)acrylate, 2-hydroxypropyl (meth)acrylate, methoxypolyethylene glycol (meth)acrylate, polyethylene glycol mono(meth)acrylate, vinylpyridine, N-vinylpyrrolidone, N-acryloylpiperidine, and N-acryloylpyrrolidine; cationic unsaturated monomers such as N,N-dimethylaminoethyl (meth)acrylate, N,N-diethylaminoethyl (meth)acrylate, N,N-dimethylaminopropyl (meth)acrylate, N,N-dimethylaminopropyl (meth)acrylamide, and quaternary salts thereof; linear celluloses, and poly($\gamma$-glutamic acid) .

When the water-absorbing resin contains a constitutional unit other than the water-soluble polymer, the content of the constitutional unit is preferably 90% by weight or less, more preferably 50% by weight or less, even more preferably 20% by weight or less, relative to the total amount of the constitutional unit and the water-soluble polymer used as the main component.

The water-absorbing resin may contain other additives, such as a disinfectant, a deodorant, an antimicrobial agent, a fragrance, various inorganic powders, a foaming agent, a pigment, a dye, a hydrophilic short fiber, a fertilizer, an oxidizing agent, a reducing agent, water, and salts, for the purpose of imparting various functions. The amount of such an additive can be appropriately selected by those skilled in the art.

<Method for degrading water-absorbing resin>

A method for degrading a water-absorbing resin of the present invention is characterized by including subjecting the water-absorbing resin produced by the above-described method to alkali treatment. In the step of alkali treatment, the water-absorbing resin is preferably placed at pH 9 or more, more preferably pH 10 or more. The alkali treatment can

cleave the crosslinked structure and glucoside bonds of the water-absorbing resin to degrade it into water-soluble polymers, thereby reducing the environmental burden at the time of disposal.

Examples of the alkaline agent used for alkali treatment include, but are not limited to, sodium hydroxide, potassium hydroxide, lithium hydroxide, ammonia, sodium carbonate, and potassium carbonate. The alkali treatment can be performed at any temperature, for example, at 5°C to 50°C.

<<Article containing water-absorbing resin>>

Various articles can be produced from the water-absorbing resin described above. Examples of the articles include hygiene and medical products such as paper diapers, sanitary products, incontinence pads, portable toilets, disposal bags, animal waste disposal agents, medical treatment materials, surgical sheets, dental waste disposal agents, medical waste blood coagulating agents, wound dressings, and moisturizing agents; agricultural and gardening products such as soil water retention agents, seedling sheets, seed coatings, sustained release agents for fertilizers, disintegration aids for agricultural chemicals and fertilizers, desert greening materials, agricultural films, and freshness-keeping agents; civil engineering materials such as soil modifiers, sludge solidifying agents, and water blocking materials; as well as articles such as refrigerants, thickening agents, deodorants, drip absorbing agents for food, pet sheets, disposable body warmers, gelling agents for batteries, dew condensation preventing sheets, packing materials, and artificial snow. Examples of the hygiene products include laminates having a back sheet, an absorber, and a top sheet stacked in this order. The absorber contains the water-absorbing resin of the present invention and may further contain a water-absorbing paper or pulp, as necessary. The specific methods for producing these articles are not limited, and each article can be produced by a well-known method.

These articles are characterized by being easily degraded because they contain the above-described water-absorbing resin. The water-absorbing resin can be degraded under the alkali treatment conditions described above, thereby reducing the environmental burden at the time of disposal.

EXAMPLES

Hereinafter, the present invention will be described in detail with reference to examples, but the present invention is not limited thereto. Hereinafter, "part(s)" and "%" mean "part(s) by weight" and "% by weight" respectively, unless otherwise specified.

(1) Materials used

(1-1) Raw material for starch

Corns starch
Tapioca
Waxy corn
Potato

(1-2) Hydrolase

α-amylase (Spitase® HK/R available from Nagase ChemteX Corporation), 12,200 units/g
Branching enzyme (Branchzyme® available from Novozymes A/S), 25,000 units/g
Amylomaltase: *Thermus thermophilus* was aerobically cultured. The homogenate extract of the collected bacterial cells was centrifuged, and the supernatant was used as a crude enzyme solution. The crude enzyme solution was subjected to column chromatography in a conventional manner, and the sample purified electrophoretically to a single product was used as a purified enzyme solution.

Here, the activity of amylomaltase was determined in the following manner. A reaction liquid 1 containing 10 w/v % maltotriose, a 50 mM sodium acetate buffer (pH 6.0), and the enzyme was incubated at 60°C for 20 minutes. Thereafter, the reaction was stopped by heating at 100°C for 10 minutes. The amount of glucose in the reaction liquid was measured by a glucose oxidase method. For the unit amount of amylomaltase, 1 unit was defined as the activity of amylomaltase that produces 1 μmol of glucose per minute.

(2) Production of partially degraded starch (Production Examples 1 to 17 and Production Example 54)

Partially degraded starches were produced in the following manner. Here, the weight average molecular weight of each resulting partially degraded starch was determined by aqueous size exclusion chromatography relative to a calibration curve of molecular weight vs. elution time prepared using pullulans having known molecular weights.

Production Example 1: Partially degraded starch derived from corn starch

Corn starch was suspended in a 20 mM acetate buffer (pH 6.0) until the concentration reached 150 (w/w) to prepare a starch milk. To the starch milk were added 0.8 units of crude enzyme solution of amylomaltase per gram of starch solids and 20 units of branching enzyme per gram of starch solids. The mixture was stirred at room temperature for 30 minutes, and then kept at 80°C for 6 hours while stirring for reaction to prepare a liquefied starch. The obtained partially degraded starch had a weight average molecular weight of 810,000.

Production Example 2: Partially degraded starch derived from corn starch

Corn starch was suspended in a 20 mM acetate buffer (pH 6.0) until the concentration reached 150 (w/w) to prepare a starch milk. To the starch milk were added 1.6 units of crude enzyme solution of amylomaltase per gram of starch solids. The mixture was stirred at room temperature for 30 minutes, and thereafter kept at 90°C for 3 hours and then at 80°C for 17 hours while stirring for reaction to prepare a liquefied starch. The obtained partially degraded starch had a weight average molecular weight of 663,000.

Production Example 3: Partially degraded starch derived from corn starch

Corn starch was suspended in a 20 mM acetate buffer (pH 6.0) until the concentration reached 150 (w/w) to prepare a starch milk. To the starch milk were added 1.6 units of purified enzyme solution of amylomaltase per gram of starch solids. The mixture was stirred at room temperature for 30 minutes, and then kept at 80°C for 8 hours while stirring for reaction to prepare a liquefied starch. The obtained partially degraded starch had a weight average molecular weight of 720,000.

Production Example 4: Partially degraded starch derived from corn starch

Corn starch was suspended in a 20 mM acetate buffer (pH 6.0) until the concentration reached 150 (w/w) to prepare a starch milk. To the starch milk were added 0.6 units of purified enzyme solution of amylomaltase per gram of starch solids and 0.03 units of $\alpha$-amylase per gram of starch solids. The mixture was stirred at room temperature for 30 minutes, and then kept at 90°C for 4.5 hours and then at 100°C for 1.5 hours while stirring for reaction to prepare a liquefied starch. The obtained partially degraded starch had a weight average molecular weight of 1,460,000.

Production Example 5: Partially degraded starch derived from tapioca starch

Tapioca starch was suspended in a 20 mM acetate buffer (pH 6.0) until the concentration reached 150 (w/w) to prepare a starch milk. To the starch milk were added 1.6 units of crude enzyme solution of amylomaltase per gram of starch solids. The mixture was stirred at room temperature for 30 minutes, and then kept at 80°C for 20 hours while stirring for reaction to prepare a liquefied starch. The obtained partially degraded starch had a weight average molecular weight of 200,000.

Production Example 6: Partially degraded starch derived from tapioca starch

Tapioca starch was suspended in a 20 mM acetate buffer (pH 6.0) until the concentration reached 150 (w/w) to prepare a starch milk. To the starch milk were added 0.4 units of crude enzyme solution of amylomaltase per gram of starch solids. The mixture was stirred at room temperature for 30 minutes, and then kept at 80°C for 9 hours while stirring for reaction to prepare a liquefied starch. The obtained partially degraded starch had a weight average molecular weight of 1,180,000.

Production Example 7: Partially degraded starch derived from tapioca starch

Tapioca starch was suspended in a 20 mM acetate buffer (pH 6.0) until the concentration reached 150 (w/w) to prepare a starch milk. To the starch milk were added 0.4 units of purified enzyme solution of amylomaltase per gram

of starch solids. The mixture was stirred at room temperature for 30 minutes, and then kept at 80°C for 3 hours while stirring for reaction to prepare a liquefied starch. The obtained partially degraded starch had a weight average molecular weight of 1,960,000.

Production Example 8: Partially degraded starch derived from waxy corn starch

Waxy corn starch was suspended in tap water, calcium chloride was added thereto until the final concentration reached 1 mM, and the pH was adjusted to 6.0 to prepare a starch milk with a concentration of about 15% by mass. To the starch milk were added 0.78 units of α-amylase per gram of starch solids. The mixture was stirred for 30 minutes and then passed through a continuous liquefaction device at a flow rate of 1 L/min. The starch milk was heated in the continuous liquefaction device at 100°C for 25 minutes, and then at 140°C for 5 minutes to prepare a liquefied starch. The obtained partially degraded starch had a weight average molecular weight of 610,000.

Production Example 9: Partially degraded starch derived from waxy corn starch

The preparation was performed in the same manner as in Production Example 8, except that the amount of enzyme added was changed to 0.36 U. The obtained partially degraded starch had a weight average molecular weight of 1,330,000.

Production Example 10: Partially degraded starch derived from potato starch

Potato starch was suspended in a 20 mM acetate buffer (pH 6.0) until the concentration reached 150 (w/w) to prepare a starch milk. To the starch milk were added 0.8 units of purified enzyme solution of amylomaltase per gram of starch solids. The mixture was stirred at room temperature for 30 minutes, and then kept at 80°C for 4 hours while stirring for reaction to prepare a liquefied starch. The obtained partially degraded starch had a weight average molecular weight of 1,530,000.

Production Example 11: Partially degraded starch derived from corn starch

Corn starch was suspended in a 20 mM acetate buffer (pH 6.0) until the concentration reached 150 (w/w) to prepare a starch milk. To the starch milk were added 0.6 units of purified enzyme solution of amylomaltase per gram of starch solids and 0.03 units of α-amylase per gram of starch solids. The mixture was stirred at room temperature for 30 minutes, and then kept at 90°C for 4.5 hours and then at 100°C for 1.5 hours while stirring for reaction to prepare a liquefied starch. The obtained partially degraded starch had a weight average molecular weight of 1,460,000.

Production Example 12: Partially degraded starch derived from corn starch

Corn starch was suspended in a 20 mM acetate buffer (pH 6.0) until the concentration reached 150 (w/w) to prepare a starch milk. To the starch milk were added 0.6 units of purified enzyme solution of amylomaltase per gram of starch solids and 0.03 units of α-amylase per gram of starch solids. The mixture was stirred at room temperature for 30 minutes, and then kept at 80°C for 6 hours while stirring for reaction to prepare a liquefied starch. The obtained partially degraded starch had a weight average molecular weight of 1,040,000.

Production Example 13: Partially degraded starch derived from tapioca starch

Tapioca starch was suspended in a 20 mM acetate buffer (pH 6.0) until the concentration reached 150 (w/w) to prepare a starch milk. To the starch milk were added 1.6 units of purified enzyme solution of amylomaltase per gram of starch solids. The mixture was stirred at room temperature for 30 minutes, and then kept at 80°C for 20 hours while stirring for reaction to prepare a liquefied starch. The obtained partially degraded starch had a weight average molecular weight of 190,000.

Production Example 14: Partially degraded starch derived from corn starch

Corn starch was suspended in a 20 mM acetate buffer (pH 6.0) until the concentration reached 150 (w/w) to prepare a starch milk. To the starch milk were added 8.0 units of purified enzyme solution of amylomaltase per gram of starch solids. The mixture was stirred at room temperature for 30 minutes, and then kept at 90°C for 3.0 hours and then at 80°C for 21 hours while stirring for reaction to prepare a liquefied starch. The obtained partially degraded starch had a weight average molecular weight of 270,000.

(Production Example 15) Partially degraded starch derived from corn starch

Corn starch was suspended in city water until the concentration reached 30% (w/w), and 1N sodium hydroxide was then added thereto to adjust the pH to 6.0 to obtain a starch milk. To the starch milk were added 0.2 units of crude enzyme solution of amylomaltase per gram of starch solids. The mixture was stirred at room temperature for 30 minutes, and then allowed to react at 80°C for 6 hours while stirring to prepare a liquefied starch. The obtained partially degraded starch had a weight average molecular weight of 6,070,000 and a dispersity of 35.4.

(Production Example 16)

Corn starch was suspended in city water until the concentration reached 30% (w/w), and 1N sodium hydroxide was then added thereto to adjust the pH to 6.0 to obtain a starch milk. To the starch milk were added 0.15 units of crude enzyme solution of amylomaltase per gram of starch solids and 0.04 units of $\alpha$-amylase per gram of starch solids. The mixture was stirred at room temperature for 30 minutes, and then allowed to react at 80°C for 6 hours while stirring to prepare a liquefied starch. The obtained partially degraded starch had a weight average molecular weight of 1,340,000 and a dispersity of 10.6. The resulting aqueous solution of the partially degraded starch was dried in vacuum at 80°C, and the resulting dried product was pulverized to collect a dry powder product of 1 mm pass. The moisture content of the dry powder product was 2.7%.

(Production Example 17)

Corn starch was suspended in city water until the concentration reached 150 (w/w), and 1N sodium hydroxide was then added thereto to adjust the pH to 6.0 to obtain a starch milk. To the starch milk were added 0.5 units of $\alpha$-amylase per gram of starch solids. The mixture was stirred at room temperature for 30 minutes, and then allowed to react at 100°C for 20 minutes while stirring to prepare a liquefied starch. The obtained partially degraded starch had a weight average molecular weight of 1,370,000 and a dispersity of 65.6.

(Production Example 54)

Corn starch was suspended in city water until the concentration reached 30% (w/w), and 1N sodium hydroxide was then added thereto to adjust the pH to 6.0 to obtain a starch milk. To the starch milk were added 0.2 units of crude enzyme solution of amylomaltase per gram of starch solids. The mixture was stirred at room temperature for 30 minutes, and then allowed to react at 80°C for 6 hours while stirring to prepare a liquefied starch. The obtained partially degraded starch had a weight average molecular weight of 1,910,000 and a dispersity of 10.1.

(3) Production of water-soluble polymer (Production Examples 18 to 34 and Production Example 55)

(3-1) Production Example 18

A 125 g portion of a 15% by weight aqueous solution of the partially degraded starch produced in Production Example 1 (0.35 mol of hydroxyl groups in the partially degraded starch) was charged into a 500 ml separable flask equipped with a stirrer, a thermometer, and a condenser tube. Next, 42.7 g of a 48.8% aqueous sodium hydroxide solution (0.52 mol, 1.5 equivalents relative to the hydroxyl group of the partially degraded starch) was charged into the flask, and stirred at a temperature of 60°C or less until the solution was completely homogeneous. After confirming that the solution had been homogeneous, an aqueous solution prepared by dissolving 60.7 g of sodium monochloroacetate (0.52 mol, 1.5 equivalents relative to the hydroxyl group of the partially degraded starch) in 78.4 g of ion-exchanged water was charged dropwise at 50°C to 60°C over 30 minutes. After charging the aqueous sodium monochloroacetate solution, the temperature was adjusted to 80°C to 85°C, and the mixture was stirred for 1 hour. The end point of the reaction was determined by sampling the reaction liquid and measuring the chlorine ion content in the reaction liquid by potentiometric titration with a 0.01 N silver nitrate solution, provided that the chlorine ion content had reached at least 98% of the calculated chlorine ion content of 6.0% which corresponds to the chloride ion content when all sodium monochloroacetate has reacted. In this production example, the chlorine content was 6.2%.

After completion of the reaction, the reaction liquid was diluted with 28 g of ion-exchanged water. The diluted reaction liquid was cooled to room temperature and added to 1 L of methanol over about 30 minutes to reprecipitate a water-soluble polymer. After adding all the reaction liquid, the mixture was stirred for 30 minutes, and the water-soluble polymer dispersed in methanol was subjected to solid-liquid separation by filtration under reduced pressure.

Subsequently, in order to remove the sodium chloride contained in the water-soluble polymer, the collected water-soluble polymer was redispersed in 0.7 L of hydrous methanol with a methanol/water ratio of 80/20 (by volume),

and then stirred and washed at room temperature for 30 minutes, followed by performing solid-liquid separation by filtration under reduced pressure to collect the water-soluble polymer again. The chlorine content of the collected water-soluble polymer was measured by potentiometric titration with a 0.01 N silver nitrate solution, and the washing process was repeated until the chlorine content was less than 1%. The resulting water-soluble polymer had a total acid value of 182 mg KOH/g and the degree of etherification calculated from the total acid value was 0.71.

(3-2) Production Examples 19 to 32

Water-soluble polymers were obtained by performing the reaction in the same manner as in Production Example 18, except that the raw materials and the amounts thereof charged were changed as shown in Table 1.

[Table 1]

| Water-soluble polymer | | Production Example 18 | Production Example 19 | Production Example 20 | Production Example 21 | Production Example 22 | Production Example 23 | Production Example 24 | Production Example 25 | Production Example 26 | Production Example 27 | Production Example 28 | Production Example 29 | Production Example 30 | Production Example 31 | Production Example 32 |
|---|---|---|---|---|---|---|---|---|---|---|---|---|---|---|---|---|
| Partially hydrolyzed starch 1 | | Production Example 1 | Production Example 2 | Production Example 3 | Production Example 4 | Production Example 5 | Production Example 6 | Production Example 7 | Production Example 7 | Production Example 8 | Production Example 8 | Production Example 9 | Production Example 10 | Production Example 11 | Production Example 12 | Production Example 15 |
| | Concentration of aqueous solution (%) | 15 | 15 | 15 | 15 | 15 | 15 | 15 | 15 | 25 | 25 | 25 | 15 | 15 | 15 | 30 |
| | Amount of aqueous solution (g) | 125.0 | 115.0 | 125.0 | 125.0 | 100.0 | 96.4 | 125.0 | 114.8 | 82.5 | 95.2 | 93.7 | 115.0 | 112.5 | 77 | 66.3 |
| Partially hydrolyzed starch 2 | | - | - | - | - | - | - | - | Production Example 13 | - | - | - | - | Production Example 14 | - | - |
| | Concentration of aqueous solution (%) | - | - | - | - | - | - | - | 15 | - | - | - | - | 15 | - | - |
| | Amount of aqueous solution (g) | - | - | - | - | - | - | - | 12.8 | - | - | - | - | 12.5 | - | - |
| Alkaline agent | 48.8% aqueous NaOH solution (g) | 42.7 | 27.5 | 42.7 | 42.7 | 23.9 | 32.9 | 42.7 | 43.6 | 5.5 | 47.4 | 53.4 | 27.5 | 29.9 | - | 23.4 |
| | 48% aqueous KOH solution (g) | - | - | - | - | - | - | - | - | - | - | - | - | - | 75 | - |

(continued)

| Water-soluble polymer | | Production Example 18 | Production Example 19 | Production Example 20 | Production Example 21 | Production Example 22 | Production Example 23 | Production Example 24 | Production Example 25 | Production Example 26 | Production Example 27 | Production Example 28 | Production Example 29 | Production Example 30 | Production Example 31 | Production Example 32 |
|---|---|---|---|---|---|---|---|---|---|---|---|---|---|---|---|---|
| Aqueous solution containing ionic functional group-containing compound | Na monochloroacetate (g) | 60.7 | 44.7 | 60.7 | 60.7 | 38.9 | 46.8 | 60.7 | 61.9 | 8.9 | 77.0 | 75.8 | 44.7 | 48.5 | - | - |
| | Monochloroacetic acid (g) | - | - | - | - | - | - | - | - | - | - | - | - | - | 30.3 | 13.0 |
| | Water for dissolving ionic functional group-containing compound (g) | 78.4 | 63.9 | 78.4 | 78.4 | 55.6 | 59.7 | 78.4 | 79.9 | 10.4 | 150.0 | 150.0 | 63.9 | 69.4 | 40.4 | 3.3 |
| Water for diluting reaction liquid (g) | | 28 | 0 | 150 | 150 | 0 | 25 | 28 | 125 | 73 | 0 | 0 | 0 | 80 | 77 | 200 |
| Methanol for reprecipitation (L) | | 1.0 | 0.8 | 1.5 | 1.5 | 0.7 | 0.8 | 1.0 | 1.4 | 0.6 | 1.0 | 1.0 | 0.8 | 1.2 | 1.0 | 1.0 |
| Hydrous methanol for washing (L) | | 0.7 | 0.6 | 0.7 | 1.3 | 0.5 | 0.5 | 0.7 | 0.8 | 0.3 | 0.5 | 0.5 | 0.7 | 0.7 | 0.7 | 0.5 |

(Production Example 33)

A 20.0 g portion of the powder of the partially degraded starch produced in Production Example 16 (0.36 mol of hydroxyl groups in the partially degraded starch) and 110 g of dimethyl sulfoxide (DMSO) were charged and dissolved in a 300 ml separable flask equipped with a stirrer, a thermometer, and a condenser tube. Next, 10.6 g of maleic anhydride (0.11 mol, 0.30 equivalents relative to the hydroxyl group of the partially degraded starch) was charged into the flask and stirred at 90°C to 95°C for 3 hours for reaction. After completion of the stirring, the reaction liquid was sampled and subjected to neutralization titration with 0.1N NaOH. The reaction liquid was found to have an acid value of 70 mg KOH/g (theoretical end point acid value: 43 mg KOH/g).

After completion of the reaction, the reaction liquid was added to 2.5 L of acetone over about 30 minutes to reprecipitate a water-soluble polymer. After adding all the reaction liquid, the mixture was stirred for 30 minutes, and the water-soluble polymer dispersed in acetone was subjected to solid-liquid separation by filtration under reduced pressure, and then collected.

Subsequently, in order to remove the unreacted maleic anhydride contained in the water-soluble polymer and the maleic acid generated by hydrolysis, the collected water-soluble polymer was redispersed in 500 mL of acetone and then stirred and washed at room temperature for 30 minutes, followed by performing solid-liquid separation by filtration under reduced pressure to collect the water-soluble polymer again. The resulting water-soluble polymer had a total acid value of 90 mg KOH/g. The resulting water-soluble polymer had a weight average molecular weight of $3.1 \times 10^6$ and a dispersity of 39.6.

(Production Example 34)

A 27.2 g portion of the powder of the partially degraded starch produced in Production Example 17 (0.47 mol of hydroxyl groups in the partially degraded starch) and 58.1 g of dimethyl sulfoxide (DMSO) were charged and dissolved in a 300 ml separable flask equipped with a stirrer, a thermometer, and a condenser tube. Next, 14.2 g of succinic anhydride (0.14 mol, 0.30 equivalents relative to the hydroxyl group of the partially degraded starch) was charged into the flask and stirred at 70°C to 75°C for 1 hours for reaction. After completion of the stirring, the reaction liquid was sampled and subjected to neutralization titration with 0.1N NaOH. The reaction liquid was found to have an acid value of 85 mg KOH/g (theoretical end point acid value: 80.1 mg KOH/g).

After completion of the reaction, the reaction solution was diluted by adding 125 g of ion-exchanged water. Further, 10.8 g (0.13 mol) of a 48% aqueous NaOH solution was added thereto to neutralize 93% of the theoretical amount of the carboxylic acid introduced by the reaction with succinic anhydride to form a sodium salt. Subsequently, the resulting solution was added to 750 ml of methanol over about 30 minutes to reprecipitate a water-soluble polymer. After adding all the reaction liquid, the mixture was stirred for 30 minutes, and the water-soluble polymer dispersed in methanol was subjected to solid-liquid separation by filtration under reduced pressure, and then collected.

Subsequently, in order to remove the unreacted succinic anhydride contained in the water-soluble polymer and the succinic acid generated by hydrolysis, the collected water-soluble polymer was redispersed in 500 mL of methanol, and then stirred and washed at room temperature for 30 minutes, followed by performing solid-liquid separation by filtration under reduced pressure to collect the water-soluble polymer again. The total acid value and free acid value of the resulting water-soluble polymer were measured. It was confirmed that the polymer had a total acid value of 138 mg KOH/g and a free acid value of 37 mg KOH/g and thus had been partially neutralized. The resulting water-soluble polymer had a weight average molecular weight of $3.7 \times 10^6$ and a dispersity of 13.8.

[Table 2]

| Water-soluble polymer | | Production Example 33 | Production Example 34 |
|---|---|---|---|
| Partially hydrolyzed starch 1 | | Production Example 16 | Production Example 17 |
| | Weight of powder (g) | 20 | 27.2 |
| DMSO(g) | | 110 | 58.1 |
| Acid anhydride (g) | Succinic anhydride | - | 14.2 |
| | Maleic anhydride | 106 | - |
| Reaction temperature (°C) | | 90-95 | 70-75 |
| Water for diluting reaction liquid (g) | | - | 125 |

(continued)

| Water-soluble polymer | | Production Example 33 | Production Example 34 |
|---|---|---|---|
| 48% aqueous NaOH solution (g) | | - | 10.8 |
| Solvent for reprecipitation (L) | Methanol | - | 0.75 |
| | Acetone | 2.5 | - |
| Solvent for washing (L) | Methanol | - | 0.5 |
| | Acetone | 0.5 | - |
| Physical properties of water-soluble polymer | Mw($\times 10^6$) | 3.1 | 3.7 |
| | Mw/Mn | 39.6 | 13.8 |
| | Total acid value (mg KOH/g) | 90 | 138 |
| | Free acid value (mg KOH/g) | - | 37 |

(Production Example 55)

A 200 g portion of a 30% by weight aqueous solution of the partially degraded starch produced in Production Example 54 (1.11 mol of hydroxyl groups in the partially degraded starch) was charged into a 500 ml separable flask equipped with a stirrer, a thermometer, and a condenser tube. Next, 79.4 g of a 48.8% aqueous sodium hydroxide solution (0.96 mol, 0.86 equivalents relative to the hydroxyl group of the partially degraded starch) was charged into the flask and then stirred at a temperature of 60°C or less until the solution became completely homogeneous. After confirming that the solution had been homogeneous, 89.9 g of crystals of 6-bromohexanoic acid (0.46 mol, 0.4 equivalents relative to the hydroxyl group of the partially degraded starch) were charged in portions at 50°C to 60°C over 30 minutes. After charging 6-bromohexanoic acid, the temperature was adjusted to 45°C to 50°C and the mixture was stirred for 10 hours. The end point of the reaction was determined by sampling the reaction liquid and measuring the bromide ion content in the reaction liquid by potentiometric titration with a 0.01 N silver nitrate solution, provided that the bromide ion content had reached at least 98% of the calculated bromide ion content of 9.8% obtained when all 6-bromohexanoic acid has reacted. In this production example, the bromine content was 10.1%.

After completion of the reaction, the reaction liquid was diluted with 250 g of ion-exchanged water. The diluted reaction liquid was cooled to room temperature and added to 1.6 L of ethanol over about 30 minutes to reprecipitate a water-soluble polymer. After adding all the reaction liquid, the mixture was stirred for 30 minutes, and the water-soluble polymer dispersed in ethanol was subjected to solid-liquid separation by filtration under reduced pressure.

Subsequently, in order to remove the sodium bromide contained in the water-soluble polymer, the collected water-soluble polymer was redispersed in 0.5 L of hydrous ethanol with an ethanol/water ratio of 90/10 (by volume), and then stirred and washed at room temperature for 30 minutes, followed by performing solid-liquid separation by filtration under reduced pressure to collect the water-soluble polymer again. The bromine content of the collected water-soluble polymer was measured by potentiometric titration with a 0.01 N silver nitrate solution, and the washing process was repeated until the bromide ion content reached less than 1%. The resulting water-soluble polymer had a total acid value of 145 mg KOH/g and the degree of etherification calculated from the total acid value was 0.64.

(4) Production of water-absorbing resin (Production Examples 35 to 51)

A 35 g portion of the water-soluble polymer obtained in Production Example 18 (product wet with hydrous methanol; wet ratio: 65%) was introduced into a 300 ml beaker, and 100 ml of hydrous methanol with a methanol/water ratio = 80/20 (by volume) was further added thereto to disperse the water-soluble polymer. To this dispersion, 3.7 ml of 1N hydrochloric acid was gradually added with a measuring pipette while stirring with a magnetic stirrer. After adding the hydrochloric acid, the mixture was stirred for 15 minutes and subjected to suction filtration under reduced pressure to collect the water-soluble polymer partially neutralized to a free acid. The collected water-soluble polymer was wet crystals containing a hydrous alcohol. The wet crystals were transferred to a petri dish, introduced into a fan dryer set at 70°C, and dried for 12 hours to perform crosslinking treatment. During the drying, methanol initially evaporated, and the water-soluble polymer once dissolved in water and became syrupy. Thus, after completion of the drying, it formed

one spongy solid mass. The spongy solid was pulverized in a mortar and sieved through sieves with openings of 150 μm and 850 μm to collect particles having a particle size of 150 to 850 μm.

The water-absorbing resins of Production Examples 36 to 51 were obtained by performing the same procedure as in Production Example 35, except that the raw materials and the amounts thereof charged were changed as shown in Table 3.

[Table 3]

| Water-absorbing resin | Production Example 35 | Production Example 36 | Production Example 37 | Production Example 38 | Production Example 39 | Production Example 40 | Production Example 41 | Production Example 42 | Production Example 43 | Production Example 44 | Production Example 45 | Production Example 46 | Production Example 47 | Production Example 48 | Production Example 49 | Production Example 50 | Production Example 51 |
|---|---|---|---|---|---|---|---|---|---|---|---|---|---|---|---|---|---|
| Water-soluble polymer | Production Example 18 | Production Example 19 | Production Example 20 | Production Example 21 | Production Example 21 | Production Example 22 | Production Example 23 | Production Example 24 | Production Example 24 | Production Example 25 | Production Example 26 | Production Example 27 | Production Example 28 | Production Example 29 | Production Example 30 | Production Example 31 | Production Example 32 |
| Weight (g) | 35 | 45 | 50 | 27 | 25 | 45 | 45 | 35 | 35 | 25 | 45 | 45 | 45 | 45 | 20 | 25 | 25 |
| Wet ratio (%) | 65 | 69 | 69 | 64 | 64 | 66 | 66 | 65 | 65 | 67 | 68 | 63 | 73 | 60 | 69 | 66 | 66 |
| Hydrous methanol (ml) | 100 | 120 | 125 | 123 | 122 | 120 | 123 | 123 | 122 | 123 | 122 | 121 | 123 | 121 | 123 | 122 | 83 |
| 1N hydrochloric acid (ml) | 37 | 4.9 | 23 | 1.7 | 3.1 | 4.8 | 2.3 | 24 | 3.5 | 1.8 | 30 | 4.3 | 2.5 | 3.6 | 1.6 | 2.8 | 1.0 |

(Production Example 52)

A 20 g portion of the water-soluble polymer obtained in Production Example 33 (product wet with hydrous acetone; wet ratio: 61%) was introduced into a 300 ml beaker, and 60 ml of hydrous acetone with an acetone /water ratio = 90/10 (by volume) was further added thereto to disperse the water-soluble polymer. To this dispersion, 19.3 ml of 1N NaOH was gradually added with a measuring pipette while stirring with a magnetic stirrer. After adding the aqueous NaOH solution, the mixture was stirred for 15 minutes and subjected to suction filtration under reduced pressure to collect the water-soluble polymer in which some carboxylic acids introduced by maleic anhydride were neutralized to a sodium salt. The collected water-soluble polymer was wet crystals containing hydrous acetone. The wet crystals were transferred to a petri dish, introduced into a fan dryer set at 70°C, and dried for 12 hours to perform crosslinking treatment. The solid obtained after the treatment was pulverized in a mortar and sieved through sieves with openings of 150 μm and 850 μm to collect particles having a particle size of 150 to 850 μm.

(Production Example 53)

A 25 g portion of the water-soluble polymer (wet crystals) obtained in Production Example 34 (product wet with hydrous methanol; wet ratio: 63%) was transferred to a petri dish, introduced into a fan dryer set at 70°C, and dried for 12 hours to perform crosslinking treatment. The solid obtained after the treatment was pulverized in a mortar and sieved through sieves with openings of 150 μm and 850 μm to collect particles having a particle size of 150 to 850 μm.

(Production Example 56)

A 25 g portion of the water-soluble polymer obtained in Production Example 55 (product wet with hydrous ethanol; wet ratio: 61%) was introduced into a 300 ml beaker, and 80 ml of hydrous ethanol with an ethanol/water ratio = 90/10 (by volume) was further added thereto to disperse the water-soluble polymer. To this dispersion, 12.1 ml of 1N hydrochloric acid was gradually added with a measuring pipette while stirring with a magnetic stirrer. After adding the hydrochloric acid, the mixture was stirred for 15 minutes and subjected to suction filtration under reduced pressure to collect the water-soluble polymer partially neutralized to a free acid. The collected water-soluble polymer was wet crystals containing a hydrous alcohol. The wet crystals were transferred to a petri dish, introduced into a fan dryer set at 150°C, and dried for 1 hour to perform crosslinking treatment. The solid obtained after the treatment was pulverized in a mortar and sieved through sieves with openings of 150 μm and 850 μm to collect particles having a particle size of 150 to 850 μm.

(5) Production of water-absorbing resin (Comparative Production Examples 1 and 2)

A water-absorbing resin of Comparative Production Example 1 was produced according to the procedure disclosed in Example 2 of Patent Literature 1 (U.S. Pat. No. 5,079,354) by carboxymethylating a corn starch whose molecular weight had not been reduced, and heat drying it under alkaline conditions.

A water-absorbing resin (hydrochloric acid-treated resin) of Comparative Production Example 2 was produced according to the procedure disclosed in Example 1 (paragraphs [0146] to [0147]) of Patent Literature 2 (JP 2010-504414 T) by crosslinking with epichlorohydrin a potato starch whose molecular weight had not been reduced, followed by carboxymethylation.

(6) Viscosity evaluation method for partially degraded starch

The viscosity of each partially degraded starch when subjected to a process of mixing with an aqueous NaOH solution was evaluated according to the following criteria. The mixing process was performed with a 500 ml separable flask set with a mechanical stirrer (three-one motor BL600 available from Shinto Scientific Co., Ltd.) with a glass anchor-type stirring blade (blade diameter: 60 mm) at the charging ratio described for each of Production Examples 15 to 28. The evaluation results are shown in Table 4.

Good: The liquid had a low viscosity and was able to be mixed homogeneously.
Average: The liquid had a high viscosity or the liquid was increased in viscosity during the mixing, so that it was not able to be mixed homogeneously.
Poor: The liquid had a high viscosity or the liquid was increased in viscosity during the mixing, so that it was not able to be mixed.

(7) Evaluation method for water-soluble polymer

(7-1) Total acid value of water-soluble polymer

A method for measuring the total acid value of a water-soluble polymer having a carboxymethyl group as an acidic group will be now described. About 0.3 g of the water-soluble polymer was precisely weighed into a 100 ml beaker and dissolved in 40 ml of ion-exchanged water. This aqueous solution was set in a potentiometric titrator (AT-610 available from Kyoto Electronics Manufacturing Co., Ltd.) equipped with glass electrodes (C-171 available from Kyoto Electronics Manufacturing Co., Ltd.). If the sample is one in which all groups form a sodium salt, it exhibits a potential of approximately 30 mV or less at this stage. Therefore, 1N hydrochloric acid was added until the potential reached 320 mV or more to convert all carboxylic acid groups in the water-soluble polymer to a free acid (resulting in an excess of hydrochloric acid). After confirming that the potential had been 320 mV or more, neutralization titration was performed with a 0.1 N aqueous NaOH solution. In this titration, two inflection points were detected, with the first inflection point being near 220 mV and the second inflection point being near 0 to -30 mV. The former is the neutralization point of the excess hydrochloric acid in the sample and the latter is the neutralization point of the carboxylic acid in the water-soluble polymer. Therefore, the total acid value is calculated by the following equation 1:

$$\text{Total acid value (mg KOH/g)} = [\{(Vb - Va) \times 0.1 \times fa \times 56.11\} \div Sa]/(1 - wr) \quad \text{(Equation 1)}$$

wherein:

Va is the volume (ml) of 0.1N NaOH consumed up to the first inflection point;
Vb is the volume (ml) of 0.1N NaOH consumed up to the second inflection point;
fa is the titer of 0.1N NaOH;
Sa is the amount of the sample collected; and
wr is the wet ratio of the water-soluble polymer measured by the method described later.

(7-2) Degree of etherification of water-soluble polymer

It is calculated from the calculated total acid value by following equation 2:

$$\text{Degree of etherification} = (162 \times TAV) \div (56100 - 80 \times TAV) \quad \text{(Equation 2)}$$

wherein TAV is the total acid value (unit: mg KOH/g) of the water-soluble polymer.

(7-3) Free acid value of water-soluble polymer

The free acid value is the acid value defined when the crosslinking of the water-soluble polymer has been performed by acid treatment. In the examples, in which the acid treatment was successively followed by the production of a crosslinked polymer, direct quantification by titration is difficult, and therefore the value calculated as follows is defined as the free acid value. Since the acid treatment was performed with an acid having a pKa lower than the carboxylic acid of the water-soluble polymer, the amount of acid added during the acid treatment is substantially equal to the free acid value. Therefore, the free acid value is calculated by the following equation 3:

$$\text{Free acid value (mg KOH/g)} = (Vc \times N \times fb \times 56.11) \div Sb \quad \text{(Equation 3)}$$

wherein:

Vc is the volume (ml) of the acid aqueous solution used in the acid treatment;
N is the normality of the acid aqueous solution;
fb is the titer of the aqueous acid solution; and
Sb is the weight (net) of the water-soluble polymer charged in the acid treatment.

(7-4) Wet ratio of water-soluble polymer

The wet ratio refers to the rate (%) of reduction in weight relative to the initial weight of the sample when the sample is dried at a drying temperature of 130°C with a halogen moisture meter. In the examples, 0.5 to 1.0 g of the water-soluble polymer was set in a halogen moisture meter HC103 available from Mettler Toledo and measured at a drying temperature of 130°C and a switch-off criterion of 1 mg/50 seconds in % MC mode (a mode that displays MC value = (initial weight of sample - dry weight of sample) ÷ initial weight of sample × 100). The displayed MC value was taken as the wet ratio.

(8) Evaluation method for water-absorbing resin

(8-1) water absorption rate under no pressure (for physiological saline)

A 1.0 g portion of each measurement sample was put in a tea bag (20 cm long × 10 cm wide) made of a nylon mesh having an opening of 63 μm (JIS Z8801-1: 2006) and immersed in 1,000 ml of physiological saline (salt concentration: 0.9% by weight) for 3 hours without stirring. Thereafter, the tea bag was hung for 10 minutes for draining. The weight (h1) including that of the tea bag was measured, and the water retention amount was determined from the following equation. Here, the temperatures of the physiological saline used and the measurement atmosphere were each 25°C ± 2°C.

$$\mathrm{FSC}\ (g/g) = (h1) - (h2)$$

Here, (h2) is the weight of the tea bag containing no measurement sample measured by the same operation as above, and FSC is an abbreviation for Free Swell Capacity which means the free swell rate, and refers to the water absorption rate under no pressure.

(8-2) Water absorption rate under no pressure (for ion-exchanged water)

The weight (h1') including that of the tea bag after immersion was measured as described for the water absorption rate under no pressure (for physiological saline), except that 0.2 g of the measurement sample was put in the tea bag and ion-exchanged water was used instead of physiological saline. Then, the water retention amount was determined from the following equation. Here, (h2') is the weight of the tea bag containing no measurement sample measured by the same operation as above.

$$\mathrm{FSC}\ (g/g) = \{(h1') - (h2')\}/0.2$$

(8-3) Water retention rate (for physiological saline)

After measuring the water absorption rate under no pressure described above, the whole tea bag was placed in a centrifuge and dehydrated by centrifugation at 150 G for 90 seconds to remove an excess liquid component. The weight (h3) including that of the tea bag was measured, and the water retention amount was determined from the following equation.

$$\mathrm{CRC}\ (g/g) = (h3) - (h4)$$

Here, (h4) is the weight of the tea bag containing no measurement sample measured by the same operation as above, and CRC is an abbreviation for Centrifuge Retention Capacity which means the centrifuge retention capacity, and refers to the water retention rate.

(8-4) water retention rate (for ion-exchanged water)

After measuring the water absorption rate under no pressure described above, the whole tea bag was placed in a centrifuge and dehydrated by centrifugation at 150 G for 90 seconds to remove an excess liquid component. The weight (h3') including that of the tea bag was measured, and the water retention amount was determined from the following equation.

$$CRC\ (g/g) = \{(h3') - (h4')\}/0.2$$

Here, (h4') is the weight of the tea bag containing no measurement sample measured by the same operation as above.

(8-5) Alkali degradability

About 5 g of the water-absorbing gel obtained after the water retention rate test with physiological saline was introduced into a 200 ml beaker, and several drops of a 48.8% aqueous NaOH solution were added onto the gel. Thereafter, the mixture was stirred with a stainless steel spoon and spread thinly on the bottom of the beaker to visually confirm the state of dissolution. When the gel state was lost and a clear solution with no insoluble matter was formed, it was evaluated as "dissolved", and when the gel state was maintained or insoluble matter remained even partially, it was evaluated as "not dissolved".

(9) Evaluation of water-absorbing resin (Examples 1 to 20 and Comparative Examples 1 and 2)

Water absorption performance and alkali degradability were measured for the water-absorbing resins obtained in Production Examples 35 to 53, Production Example 56, and Comparative Production Examples 1 and 2. The results are shown in Table 4.

[Table 4]

| | | Ex. 1 | Ex. 2 | Ex. 3 | Ex. 4 | Ex. 5 | Ex. 6 | Ex. 7 | Ex. 8 | Ex. 9 | Ex. 10 | Ex. 11 | Ex. 12 | Ex. 13 | Ex. 14 | Ex. 15 | Ex. 16 | Ex. 17 | Ex. 18 | Ex. 19 | Ex. 20 | Comp. Ex. 1 | Comp. Ex. 2 |
|---|---|---|---|---|---|---|---|---|---|---|---|---|---|---|---|---|---|---|---|---|---|---|---|
| Water-soluble polymer | | Prod. Ex. 18 | Prod. Ex. 19 | Prod. Ex. 20 | Prod. Ex. 21 | Prod. Ex. 21 | Prod. Ex. 22 | Prod. Ex. 23 | Prod. Ex. 24 | Prod. Ex. 24 | Prod. Ex. 25 | Prod. Ex. 26 | Prod. Ex. 27 | Prod. Ex. 28 | Prod. Ex. 29 | Prod. Ex. 30 | Prod. Ex. 31 | Prod. Ex. 32 | Prod. Ex. 33 | Prod. Ex. 34 | Prod. Ex. 55 | Comp. Prod. Ex. 1 | Comp. Prod. Ex. 2 |
| Water-absorbing resin | | Prod. Ex. 35 | Prod. Ex. 36 | Prod. Ex. 37 | Prod. Ex. 38 | Prod. Ex. 39 | Prod. Ex. 40 | Prod. Ex. 41 | Prod. Ex. 42 | Prod. Ex. 43 | Prod. Ex. 44 | Prod. Ex. 45 | Prod. Ex. 46 | Prod. Ex. 47 | Prod. Ex. 48 | Prod. Ex. 49 | Prod. Ex. 50 | Prod. Ex. 51 | Prod. Ex. 52 | Prod. Ex. 53 | Prod. Ex. 56 | | |
| Type of starch | | Corn starch | Corn starch | Corn starch | Corn starch | Corn starch | Tapioca | Tapioca | Tapioca | Tapioca | Tapioca | Waxy | Waxy | Waxy | Potato | Corn starch | Corn starch | Corn starch | Corn starch | Corn starch | Corn starch | Corn starch | Potato |
| Crosslinking (acid or base) | | HCl | HCl | HCl | HCl | HCl | HCl | HCl | HCl | HCl | HCl | HCl | HCl | HCl | HCl | HCl | HCl | HCl | NaOH | NaOH | HCl | None | (Epichloro-hydrin) |
| Partially hydrolyzed starch 1 | | Prod. Ex. 1 | Prod. Ex. 2 | Prod. Ex. 3 | Prod. Ex. 4 | Prod. Ex. 4 | Prod. Ex. 5 | Prod. Ex. 6 | Prod. Ex. 7 | Prod. Ex. 7 | Prod. Ex. 7 | Prod. Ex. 8 | Prod. Ex. 8 | Prod. Ex. 9 | Prod. Ex. 10 | Prod. Ex. 11 | Prod. Ex. 12 | Prod. Ex. 15 | Prod. Ex. 16 | Prod. Ex. 17 | Prod. Ex. 54 | - | - |
| | Amount used (% by mass) | 100 | 100 | 100 | 100 | 100 | 100 | 100 | 100 | 100 | 90 | 100 | 100 | 100 | 100 | 90 | 100 | 100 | 100 | 100 | 100 | - | - |
| | Mw(×10⁶) | 0.81 | 0.66 | 0.72 | 1.46 | 1.46 | 0.20 | 1.18 | 1.96 | 1.96 | 1.96 | 0.61 | 0.61 | 1.33 | 1.53 | 1.46 | 1.04 | 6.07 | 1.34 | 1.37 | 1.91 | - | - |
| | Mw/Mn | 6.4 | 7.4 | 8.7 | 9.8 | 9.8 | 2.9 | 22.6 | 8.0 | 8.0 | 8.0 | 35.2 | 35.2 | 19.1 | 9.9 | 9.8 | 10.6 | 35.4 | 10.6 | 65.6 | 10.1 | - | - |
| Partially hydrolyzed starch 2 | | - | - | - | - | - | - | - | - | - | Prod. Ex. 13 | - | - | - | - | Prod. Ex. 14 | - | - | - | - | - | - | - |
| | Amount used (% by mass) | 0 | 0 | 0 | 0 | 0 | 0 | 0 | 0 | 0 | 10 | 0 | 0 | 0 | 0 | 10 | 0 | 0 | 0 | 0 | 0 | - | - |
| | Mw(×10⁶) | - | - | - | - | - | - | - | - | - | 0.19 | - | - | - | - | 0.27 | - | - | - | - | - | - | - |
| | Mw/Mn | - | - | - | - | - | - | - | - | - | 2.94 | - | - | - | - | 4.40 | - | - | - | - | - | - | - |
| Counter cation | | Sodium | Sodium | Sodium | Sodium | Sodium | Sodium | Sodium | Sodium | Sodium | Sodium | Sodium | Sodium | Sodium | Sodium | Sodium | Potassium | Sodium | Sodium | Sodium | Sodium | Sodium | Sodium |
| Physical properties of water-soluble polymer | Mw(×10⁶) | 3.4 | 2.0 | 2.3 | 4.0 | 4.0 | 0.8 | 5.3 | 11.5 | 11.5 | 9.4 | 1.6 | 2.3 | 4.5 | 9.3 | 3.4 | 1.9 | 45.6 | 3.1 | 3.7 | 4.8 | - | - |
| | Mw/Mn | 9.3 | 8.7 | 8.5 | 7.2 | 7.2 | 10.1 | 16.8 | 19.3 | 19.3 | 25.3 | 7.4 | 22.3 | 14.6 | 20.9 | 16.1 | 11.2 | 104.9 | 39.6 | 13.8 | 14.0 | - | - |
| | Total acid value (mg KOH/g) | 182 | 170 | 180 | 161 | 161 | 142 | 165 | 170 | 170 | 172 | 71 | 192 | 191 | 162 | 134 | 138 | 154 | 90 | 138 | 145 | - | - |
| | Degree of etherification | 0.71 | 0.65 | 0.70 | 0.60 | 0.60 | 0.51 | 0.62 | 0.65 | 0.65 | 0.66 | 0.23 | 0.76 | 0.76 | 0.61 | 0.48 | 0.50 | 0.57 | 0.32 (*1) | 0.57 (*2) | 0.64 | - | - |
| | Free acid value (mg KOH/g) | 17 | 22 | 11 | 11 | 22 | 17 | 8 | 8 | 11 | 11 | 11 | 17 | 8 | 17 | 11 | 17 | 11 | 17 | 37 | 11 | - | - |
| | Viscosity | Good | Good | Good | Good | Good | Good | Good | Good | Good | Good | Good | Good | Good | Good | Good | Good | Good | Good | Good | Good | Average | Average |
| Water absorption performance | FSC (Ion-exchanged water) | 179 | 140 | 216 | 227 | 156 | 212 | 268 | 331 | 185 | 289 | 152 | 171 | 251 | 228 | 216 | 233 | 143 | 40 | 160 | 62 | 69 | 292 |
| | CRC (Ion-exchanged water) | 147 | 112 | 131 | 160 | 127 | 154 | 172 | 186 | 152 | 184 | 122 | 130 | 145 | 182 | 160 | 140 | 112 | 21 | 63 | 39 | 21 | 81 |
| | FSC (Physiological saline) | 43 | 36 | 34 | 51 | 41 | 42 | 63 | 64 | 44 | 58 | 23 | 29 | 53 | 48 | 52 | 47 | 27 | 11 | 25 | 20 | 15 | 29 |
| | CRC (Physiological saline) | 39 | 33 | 29 | 44 | 38 | 36 | 55 | 50 | 41 | 51 | 21 | 27 | 42 | 44 | 46 | 39 | 24 | 7 | 22 | 14 | 5 | 26 |
| Alkali degradability | | Dissolved | Dissolved | Dissolved | Dissolved | Dissolved | Dissolved | Dissolved | Dissolved | Dissolved | Dissolved | Dissolved | Dissolved | Dissolved | Dissolved | Dissolved | Dissolved | Dissolved | Dissolved | Dissolved | Dissolved | Not dissolved | Not dissolved |

Ex.: Example
Comp. Ex.: Comparative Example
Prod. Ex.: Production Example
Comp. Prod. Ex.: Comparative Production Example

*1: Degree of esterification with maleic anhydride = (162*Total acid value (mg KOH/g))/(56100-120*Total acid value(mg KOH/g))
*2: Degree of esterification with succinic anhydride=(162*Total acid value(mg KOH/g))/(56100-122*Total acid value(mg KOH/g))

As shown in Table 4, the water-absorbing resins of Examples 1 to 20 were superior in water absorption performance to those of Comparative Examples 1 and 2.

Moreover, the water-soluble polymers used as raw materials in Examples 1 to 20 had a lower viscosity and better operability during the production of a water-absorbing resin than those of Comparative Examples 1 and 2. Those having a higher viscosity is more difficult to mix homogeneously, which may limit the options for production equipment and may cause variations in quality. Moreover, large dilution may be required for homogeneous mixing, leading to a reduction in production efficiency.

## Claims

1. A method for producing a water-absorbing resin, comprising:

   (c1) producing a water-soluble polymer by:

   (a1) reducing a molecular weight of a starch to obtain a partially degraded starch; and
   (a2) introducing an acidic group into the partially degraded starch obtained in the step (a1);

   (c2) neutralizing the acidic group introduced into the water-soluble polymer; and
   (c3) crosslinking one water-soluble polymer with another.

2. The method for producing a water-absorbing resin according to claim 1, wherein the acidic group is an acidic group having a carboxyalkyl group, a carboxyalkenyl group, or a sulfoalkyl group.

3. The method for producing a water-absorbing resin according to claim 1 or 2, wherein the obtained water-absorbing resin has the following characteristics:

   (a) a water absorption rate under no pressure for ion-exchanged water is 100 to 400 g/g;
   (b) a water retention rate for ion-exchanged water is 80 to 300 g/g;
   (c) a water absorption rate under no pressure for physiological saline is 20 to 70 g/g; and/or
   (d) a water retention rate for physiological saline is 7 to 60 g/g.

4. The method for producing a water-absorbing resin according to any one of claims 1 to 3, wherein the obtained water-absorbing resin has a ratio (A/B) of a water absorption rate under no pressure for ion-exchanged water (A) to a water absorption rate under no pressure for physiological saline (B) of 7 or less.

5. The method for producing a water-absorbing resin according to any one of claims 1 to 4, wherein no crosslinking agent is used in the step (c3) .

6. A method for degrading a water-absorbing resin, comprising subjecting the water-absorbing resin obtained by the method according to any one of claims 1 to 5 to alkali treatment.

7. A method for producing an article comprising the water-absorbing resin obtained by the method according to any one of claims 1 to 5.

8. A method for producing a water-soluble polymer, comprising:

   (a1) reducing a molecular weight of a starch to obtain a partially degraded starch; and
   (a2) introducing an acidic group into the partially degraded starch obtained in the step (a1).

9. The method for producing a water-soluble polymer according to claim 8, wherein the partially degraded starch obtained in the step (a1) has a weight average molecular weight (Mw) of 7,500,000 or less and/or a dispersity (weight average molecular weight (Mw)/number average molecular weight (Mn)) of 5 or more.

10. A method for producing a water-soluble polymer, comprising:

    (b1) introducing an acidic group into a starch; and
    (b2) reducing a molecular weight of the starch having the acidic group obtained in the step (b1) to obtain a partially degraded starch having the acidic group.

11. The method for producing a water-soluble polymer according to any one of claims 8 to 10, wherein in the step (a1) or (b2), the molecular weight of the starch is reduced by enzymatic treatment.

12. The method for producing a water-soluble polymer according to any one of claims 8 to 11, wherein the obtained water-soluble polymer has a weight average molecular weight (Mw) of 500,000 to 50,000,000 as determined by aqueous size exclusion chromatography using pullulan standards.

**13.** A method for producing a resin composition for producing a water-absorbing resin comprising the water-soluble polymer obtained by the method according to any one of claims 8 to 12.

**INTERNATIONAL SEARCH REPORT**

International application No. **PCT/JP2022/016403**

**A. CLASSIFICATION OF SUBJECT MATTER**

***C08B 31/12***(2006.01)i; ***C08H 99/00***(2010.01)i
FI: C08B31/12; C08H99/00

According to International Patent Classification (IPC) or to both national classification and IPC

**B. FIELDS SEARCHED**

Minimum documentation searched (classification system followed by classification symbols)

C08B31/12; C08H99/00

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

CAplus/REGISTRY (STN)

**C. DOCUMENTS CONSIDERED TO BE RELEVANT**

| Category* | Citation of document, with indication, where appropriate, of the relevant passages | Relevant to claim No. |
|---|---|---|
| X | JP 59-039300 A (WAKO PURE CHEMICAL INDUSTRIES, LTD.) 03 March 1984 (1984-03-03) example 1 | 8-10, 12 |
| Y | | 11 |
| A | | 1-7, 13 |
| X | JP 59-031699 A (WAKO PURE CHEMICAL INDUSTRIES, LTD.) 20 February 1984 (1984-02-20) example 1 | 8-10, 12 |
| Y | | 11 |
| A | | 1-7, 13 |
| X | JP 2005-075815 A (TANIHARA, Masao) 24 March 2005 (2005-03-24) example 3 | 8-10, 12 |
| Y | | 11 |
| A | | 1-7, 13 |

☑ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **15 June 2022** | **28 June 2022** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | <br>Telephone No. |

Form PCT/ISA/210 (second sheet) (January 2015)

**INTERNATIONAL SEARCH REPORT**

International application No.
**PCT/JP2022/016403**

**C. DOCUMENTS CONSIDERED TO BE RELEVANT**

| Category* | Citation of document, with indication, where appropriate, of the relevant passages | Relevant to claim No. |
|---|---|---|
| X | US 4321364 A (MINISTER FOR PUBLIC WORKS FOR THE STATE OF NEW SOUTH WALES) 23 March 1982 (1982-03-23)<br>example 1 | 8-10, 12 |
| Y | | 11 |
| A | | 1-7, 13 |
| X | CN 108456256 A (JIANGNAN UNIVERSITY) 28 August 2018 (2018-08-28)<br>examples 1-6 | 8-10, 12 |
| Y | | 11 |
| A | | 1-7, 13 |
| Y | 澱粉化学ハンドブック. Asakura Publishing Co., Ltd., 01 March 1978, pp. 520-521, non-official translation (Starch Chemistry Handbook.)<br>entire text (section 23.14) | 11 |
| A | | 1-10, 12, 13 |
| X | PFEIFFER, K. et al. Starch Derivatives of High Degree of Functionalization 5. Stepwise Carboxymethylation of Amylose. Chemical Papers. 2002, vol. 56(4), pp. 261-266<br>entire text (page 262) | 8, 9, 12 |
| A | | 1-7, 10, 11, 13 |
| A | JP 2019-056071 A (NIPPON SHOKUHIN KAKO CO., LTD.) 11 April 2019 (2019-04-11)<br>paragraph [0004] | 1-13 |
| A | entire text | 1-13 |
| A | JP 2002-504508 A (LENAERTS, Vincent) 12 February 2002 (2002-02-12)<br>entire text | 1-13 |
| A | JP 2017-523277 A (ROQUETTE ITALIA S. P. A.) 17 August 2017 (2017-08-17)<br>entire text | 1-13 |
| A | JP 2012-012462 A (INSTITUTE OF NATIONAL COLLEGES OF TECHNOLOGY JAPAN) 19 January 2012 (2012-01-19)<br>entire text | 1-13 |
| A | JP 60-055522 B2 (NICHIDEN KAGAKU KK) 05 December 1985 (1985-12-05)<br>entire text | 1-13 |
| A | JP 61-095006 A (NIPPON SHOKUHIN KAKO CO., LTD.) 13 May 1986 (1986-05-13)<br>entire text | 1-13 |

Form PCT/ISA/210 (second sheet) (January 2015)

**INTERNATIONAL SEARCH REPORT**
**Information on patent family members**

International application No.
**PCT/JP2022/016403**

| Patent document cited in search report | Publication date (day/month/year) | Patent family member(s) | Publication date (day/month/year) |
|---|---|---|---|
| JP 59-039300 A | 03 March 1984 | EP 0104780 A1<br>example 1 | |
| JP 59-031699 A | 20 February 1984 | (Family: none) | |
| JP 2005-075815 A | 24 March 2005 | (Family: none) | |
| US 4321364 A | 23 March 1982 | (Family: none) | |
| CN 108456256 A | 28 August 2018 | (Family: none) | |
| JP 2019-056071 A | 11 April 2019 | (Family: none) | |
| JP 2002-504508 A | 12 February 2002 | US 6284273 B1<br>entire text<br>US 6419957 B1<br>WO 1999/043305 A1<br>EP 1059915 A1<br>CN 1298297 A<br>KR 10-0713702 B1 | |
| JP 2017-523277 A | 17 August 2017 | US 2017/0130052 A1<br>entire text<br>WO 2016/004974 A1<br>EP 3166977 A1<br>CN 106573992 A | |
| JP 2012-012462 A | 19 January 2012 | (Family: none) | |
| JP 60-055522 B2 | 05 December 1985 | (Family: none) | |
| JP 61-095006 A | 13 May 1986 | (Family: none) | |

Form PCT/ISA/210 (patent family annex) (January 2015)

## REFERENCES CITED IN THE DESCRIPTION

*This list of references cited by the applicant is for the reader’s convenience only. It does not form part of the European patent document. Even though great care has been taken in compiling the references, errors or omissions cannot be excluded and the EPO disclaims all liability in this regard.*

### Patent documents cited in the description


- US 5079354 A
- JP 2010504414 T
- JP 2007222704 A


### Non-patent literature cited in the description


- *Synthesis,* 1989, (12), 949-950